# EUROPEAN PATENT APPLICATION

(11) **EP 3 513 665 A1**
(43) Date of publication of application: **24.07.2019**
(21) Application number: 19153200.1
(22) Date of filing: 23.01.2019
(51) Int. Cl.: A23L 33/00, A23L 33/135, A23L 33/15, A23L 33/16, A23L 33/21, A61K 31/702, A61K 33/30, A61K 35/745, A61P 1/00

(54) **DIETARY SUPPLEMENT AND USES THEREOF**

(30) Priority: 23.01.2018 BE 201800011
(71) Applicant: Omega Pharma Innovation and Development NV, 9810 Nazareth (BE)
(72) Inventor: Spooren, Anneleen, 9000 Gent (BE); Andries, Oliwia, 9000 Gent (BE)
(74) Representative: De Clercq & Partners

(57) **Abstract**

The invention concerns dietary supplement for weight loss and/or weight maintenance, wherein the dietary supplement comprises: a probiotic; a chloride salt; choline or a salt thereof; zinc or a salt thereof; vitamin B2; and a prebiotic, wherein said dietary supplement comprises no fatty acids and/or no carbohydrates in addition to fatty acids and/or carbohydrates comprised within the prebiotic and/or probiotic. The invention further relates to the use of the dietary supplement for preparing the digestive system of a subject in advance of a weight loss program and to a dietary supplement for use in a method of treating obesity, low-grade inflammation, diabetes, non-alcoholic fatty liver disease (NAFLD), metabolic endotoxemia, metabolic syndrome and/or impaired glucose tolerance in a subject.

## Description

### FIELD OF THE INVENTION

The invention is broadly in the medical field, more precisely in the field of dietary supplements. In particular, the invention concerns a dietary supplement and its use for preparing the body before a weight loss treatment.

### BACKGROUND OF THE INVENTION

Worldwide obesity has more than doubled since 1980. In 2014, more than 1.9 billion adults were overweight, of which over 600 million were obese. Obesity is not only an adult disease. Prevalence of obesity among children has also increased dramatically in the last decades. 41 million children under the age of 5 were overweight or obese in 2014, placing them also at risk for obesity into adulthood. Both in adults and children, obesity can have serious health consequences such as cardiovascular diseases, diabetes, musculoskeletal disorders and some cancers.

Numerous efforts at prevention and treatment exist to address the multifactorial causes and consequences of obesity. Weight-loss measures and programs include weight-control diets which reduce intake of calories and/or eliminate certain food groups from the diet; weight-loss products for reducing appetite, reducing adsorption of nutrients and/or increasing fat burning; physical exercises; and surgical weight loss procedures such as gastric bypass, sleeve gastrectomy, gastric balloons and gastric bands. Many of these weight-loss measures and programs may give rise to side effects such as heart problems, liver problems, kidney problems and muscle loss; others are nutritionally incomplete or have high drop-out rates.

"Dieting" is currently falling out of fashion and efforts are made to incorporate weight management in an overall healthy lifestyle.

In view thereof, there is a need for further and/or improved means to lose weight and/or to assist weight loss programs.

### SUMMARY OF THE INVENTION

The present inventors have found a dietary supplement comprising a unique combination of active ingredients for effectively preparing the body before a weight loss program. More particularly, the inventors have realised that a dietary supplement comprising the specific combination of at least one probiotic and at least one ingredient selected from an ingredient capable of cleansing toxins and protecting against their build up, an ingredient capable of supporting lipid and fatty acid metabolism, an ingredient capable of supporting healthy digestion, wherein said ingredient capable of supporting healthy digestion is an ion or a salt thereof, and an ingredient capable of supporting healthy liver function, and especially a dietary supplement comprising the specific combination of a probiotic, a prebiotic, chloride or a salt thereof, choline or a salt thereof, zinc or a salt thereof, and vitamin B2, allows to assist elimination of toxins, and to support metabolism and digestion.

Accordingly, a first aspect of the invention relates to a dietary supplement for weight loss and/or weight maintenance, wherein the dietary supplement comprises: at least one probiotic and at least one ingredient selected from:
- an ingredient capable of cleansing toxins and protecting against their build up,
- an ingredient capable of supporting lipid and fatty acid metabolism,
- an ingredient capable of supporting healthy liver function, and
- an ingredient capable of supporting healthy digestion, wherein said ingredient capable of supporting healthy digestion is an ion or a salt thereof.

In certain embodiments, the invention provides a dietary supplement for weight loss and/or weight maintenance, wherein the dietary supplement comprises a probiotic; chloride or a salt thereof; choline or a salt thereof; zinc or a salt thereof; vitamin B2; and a prebiotic.

Preferably, an aspect of the invention relates to a dietary supplement for weight loss and/or weight maintenance, wherein the dietary supplement comprises: at least one probiotic and at least one ingredient selected from:
- an ingredient capable of cleansing toxins and protecting against their build up,
- an ingredient capable of supporting lipid and fatty acid metabolism,
- an ingredient capable of supporting healthy liver function, and
- a salt capable of supporting healthy digestion.

Preferably, the invention provides a dietary supplement for weight loss and/or weight maintenance, wherein the dietary supplement comprises a probiotic; a chloride salt; choline or a salt thereof; zinc or a salt thereof; vitamin B2; and a prebiotic.

Advantageously, the present dietary supplement allows to help a subject to prepare its body in advance of a weight loss program. The dietary supplement embodying the principles of the present invention helps to cleanse toxins from the digestive system and protects against further build-up of toxins, thereby allowing the subject to "detox" or "cleanse" its body in preparation of a weight loss treatment. Further, the present dietary supplement allows to support a subject's healthy fat metabolism and to maintain healthy digestion by contributing to gut flora balance. Thereby, the present dietary supplement may improve the results of a follow-up weight loss program conducted by the subject. The present dietary supplement also has the benefit that is to be taken only once a day and can be used anytime of the day, for instance before or after meals, or in between meals.

A further aspect relates to a sachet comprising a dietary supplement as defined herein. Advantageously, the present sachet allows the subject to consume a daily dose of the dietary supplement in a straightforward manner. The present sachet can be taken along without difficulty and allows its content to be easily dissolved in water or another liquid prior to consumption.

A further aspect provides the use of a dietary supplement as defined herein for preparing the digestive system of a subject in advance of a weight loss program; for supporting the digestive system of a subject during or after a weight loss program; for supporting fat metabolism, gut barrier function and/or digestive health of a subject; for promoting elimination of toxins from the digestive system of a subject; for avoiding accumulation of toxins in the digestive system of a subject; for supporting liver function and/or kidney function of a subject; for supporting weight loss of a subject; for controlling weight gain; for improving body shape of a subject; and/or for reducing total body fat mass, trunk fat mass, android fat mass and/or energy intake of a subject. These effects may be obtained with no changes to the diet or exercise habits of the subject.

The present inventors have further realised that a dietary supplement as defined herein may have a therapeutic effect when administered to subjects having obesity, low-grade inflammation, diabetes, non-alcoholic fatty liver disease (NAFLD), metabolic endotoxemia, metabolic syndrome and/or impaired glucose tolerance.

Accordingly, a further aspect relates to the use of a dietary supplement as defined herein as a nutraceutical.

A further aspect relates to a dietary supplement as defined herein for use in a method of treating obesity, low-grade inflammation, diabetes, non-alcoholic fatty liver disease (NAFLD), metabolic endotoxemia, metabolic syndrome and/or impaired glucose tolerance in a subject.

A further aspect provides a method for producing a dietary supplement as defined herein, the method comprising the step of preparing a mixture of:
at least one probiotic and at least one ingredient selected from:
   - an ingredient capable of cleansing toxins and protecting against their build up,
   - an ingredient capable of supporting lipid and fatty acid metabolism,
   - an ingredient capable of supporting healthy liver function, and
   - an ingredient capable of supporting healthy digestion, wherein said ingredient capable of supporting healthy digestion is an ion or a salt thereof,
thereby obtaining the dietary supplement.

In certain embodiments, the invention provides a method for producing the dietary supplement as defined herein, the method comprising the step of preparing a mixture of: the probiotic; the chloride or the salt thereof; the choline or the salt thereof; the zinc or the salt thereof; and the vitamin B2; and the prebiotic, thereby obtaining the dietary supplement.

Preferably, a further aspect provides a method for producing a dietary supplement as defined herein, the method comprising the step of preparing a mixture of:
at least one probiotic and at least one ingredient selected from:
   - an ingredient capable of cleansing toxins and protecting against their build up,
   - an ingredient capable of supporting lipid and fatty acid metabolism,
   - an ingredient capable of supporting healthy liver function, and
   - a salt capable of supporting healthy digestion,
thereby obtaining the dietary supplement.

Preferably, the invention provides a method for producing the dietary supplement as defined herein, the method comprising the step of preparing a mixture of: the probiotic; the chloride salt; the choline or the salt thereof; the zinc or the salt thereof; and the vitamin B2; and the prebiotic, thereby obtaining the dietary supplement.

A further aspect provides a method for producing a sachet comprising a dietary supplement as defined herein, the method comprising the step of filling a sachet with a single dose of the dietary supplement as defined herein and closing the filled sachet.

The above and further aspects and preferred embodiments of the invention are described in the following sections and in the appended claims. The subject-matter of appended claims is hereby specifically incorporated in this specification.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of' and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All documents cited in the present specification are hereby incorporated by reference in their entirety.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The present inventors have found a dietary supplement comprising a unique combination of active ingredients for weight loss and/or weight maintenance; for weight management; and/or for effectively preparing the body before a weight loss program. More particularly, the inventors have found that the specific combination of at least one probiotic and at least one ingredient selected from an ingredient capable of cleansing toxins and protecting against their build up, an ingredient capable of supporting lipid and fatty acid metabolism, an ingredient capable of supporting healthy digestion, wherein said ingredient capable of supporting healthy digestion is an ion or a salt thereof, and an ingredient an ingredient capable of supporting healthy liver function, in particular a dietary supplement comprising the specific combination of a probiotic, chloride or a salt thereof, choline or a salt thereof, zinc or a salt thereof, vitamin B2, and a prebiotic, allows to assist elimination of toxins, and to support metabolism and digestion.

In the context of the present invention, the dietary supplement is preferably formulated as a soluble powder, even more preferably as a soluble powder suitable for oral administration.

Accordingly, a first aspect provides a dietary supplement for weight loss and/or weight maintenance; for weight management; and/or for effectively preparing the body before a weight loss program, wherein the dietary supplement comprises: at least one probiotic and at least one ingredient selected from:
- an ingredient capable of cleansing toxins and protecting against their build up,
- an ingredient capable of supporting lipid and fatty acid metabolism,
- an ingredient capable of supporting healthy digestion, wherein said ingredient capable of supporting healthy digestion is an ion or a salt thereof, and
- an ingredient capable of supporting healthy liver function.

In particular embodiments, the dietary supplement for weight loss and/or weight maintenance, as disclosed herein, comprises: at least one probiotic and at least one ingredient selected from:
- an ingredient capable of cleansing toxins and protecting against their build up,
- an ingredient capable of supporting lipid and fatty acid metabolism,
- an ingredient capable of supporting healthy liver function, and
- a salt capable of supporting healthy digestion.

The term "dietary supplement" as used herein refers to a product intended to supplement or complement a subject's diet, the product comprising one or more substances with a nutritional and/or physiological effect on a subject. The dietary supplement can be a partial nutritional composition, which does not contain all the essential macro- and micronutrients and hence may not be used to replace one or more daily meals and/or may not be used as the sole source of nutrition of a subject. The dietary supplement may be a liquid, powder, gel, paste, solid, concentrate, suspension or ready-to-use formulation.

The term "weight loss" as used herein refers to a reduction of the total body mass, which may be due to a mean loss of fluid, adipose tissue and/or lean mass. Weight loss can occur involuntary due to malnourishment or an illness or arise from a voluntary effort to improve an actual or perceived overweight or obese state.

The term "weight maintenance" as used herein refers to the process of preserving a certain total body mass over time. Weight maintenance may be defined as a change in total body mass of less than 5%, less than 4%, less than 3%, less than 2% or less than 1%, preferably less than 5% of the total body mass, over time, such as over a period of one or more months, e.g., over a period of six or more months, such as over a period of one or more years.

The term "weight management" as used herein refers to balancing a subject's energy intake and energy consumption over time in order to maintain a certain total body mass over time and/or to reduce the total body mass of the subject.

The term "weight loss program" or "weight loss plan" as used herein refers to a plan of action directed at losing weight and/or body fat. Weight loss programs may include changes in nutrition, physical activity and/or lifestyle, the ingestion of weight loss pills and/or dietary supplements, and/or surgical weight loss procedures. Non-limiting examples of nutritional changes include weight-control diets which reduce intake of calories and/or eliminate certain food groups from the diet. Non-limiting examples of weight loss pills and/or supplements include weight-loss products for reducing appetite, reducing adsorption of nutrients and/or increasing fat burning. Non-limiting examples of surgical weight loss procedures include gastric bypass, sleeve gastrectomy, gastric balloons and gastric bands. Non-limiting examples of weight loss programs include XLS-Medical weight loss plan, Weight Watchers, Atkins diet, Dukan diet, Keto diet, and Paleo diet.

The term "probiotic" as used herein, refers to microorganisms, such as bacteria and yeasts, which exert beneficial effects on the health of the host, especially on the digestive system, when consumed in adequate amounts. Such health benefits may include, but are not limited to, preventing allergies; controlling high blood pressure; reducing cholesterol levels; treating gastroenteritis, inflammatory bowel disease and/or inflammatory bowel disease; preventing skin conditions such as eczema; and improving immune function. Any probiotic known in the art may be acceptable. Such probiotics may be naturally-occurring, synthetic, or developed through the genetic manipulation of organisms. Preferably, the probiotic(s) are viable microorganisms. As used herein, the term "viable" refers to live microorganisms.

The phrase "at least one probiotic" refers to one or more probiotics, such as two or more, three of more, or four or more, such as five, six, seven, eight or more probiotics.

In certain embodiments, the probiotic may be selected from the group consisting of Bifidobacterium, Saccharomyces, Debaromyces, Candida, Pichia, Torulopsis, Aspergillus, Rhizopus, Mucor, Penicillium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, and Lactobacillus. Preferably, the probiotic is Bifidobacterium.

In certain embodiments, the probiotic may be one or more Bifidobacterium species selected from the group consisting of *Bifidobacterium animalis*, *Bifidobacterium lactis, Bifidobacterium bifidium*, *Bifidobacterium longum, Bifidobacterium breve, Bifidobacterium infantis, Bifidobacterium catenulatum*, *Bifidobacterium adolescentis*, and *Bifidobacterium angalatum*. Preferably, the probiotic is a Bifidobacterium of the species *Bifidobacterium animalis*.

In certain embodiments, the probiotic may be a Bifidobacterium of the species *Bifidobacterium animalis* subsp. *lactis* or *Bifidobacterium animalis* subsp. *Animalis*, or a combination thereof.

Preferably, the probiotic is a Bifidobacterium of the species *Bifidobacterium animalis* subsp. *Lactis.*

In certain embodiments, the probiotic may be one or more *Bifidobacterium animalis* subsp. *lactis* strains selected from the group consisting of B420, BB12, HN019, Bl-04, Bi-07, DN-173-010, BLC1, DSM10140, V9, CNCM 1-2495, AD011, ATCC 27536, NCC 2818, CNCM 1-3446 and VTT E-012010.

An exemplary *Bifidobacterium animalis* subsp. *lactis* strain B420 is the strain deposited under accession number ATCC SD6685.

An exemplary *Bifidobacterium animalis* subsp. *lactis* strain HN019, also known as CHCC7158, DR10™ or HOWARU™, is the strain deposited under accession number DSM17280.

An exemplary *Bifidobacterium animalis* subsp. *lactis* strain Bl-04, also known as DGCC2908 and RB 4825, is the strain deposited at NCBI under accession number CP001515.

An exemplary *Bifidobacterium animalis* subsp. *lactis* strain Bi-07 is the strain deposited under accession number ATCC PTA-4802.

An exemplary *Bifidobacterium animalis* subsp. *lactis* strain BLC1 is the strain deposited in GenBank under accession number CP003039.

An exemplary *Bifidobacterium animalis* subsp. *lactis* strain UR1 is the strain deposited under accession number DSM10140, CIP 105265 and JCM 10602.

An exemplary *Bifidobacterium animalis* subsp. *lactis* strain V9 is the strain deposited in GenBank under accession number CP001892.

An exemplary *Bifidobacterium animalis* subsp. *lactis* strain CNCM 1-2495 is the strain deposited in GenBank under accession number CP002915.

An exemplary *Bifidobacterium animalis* subsp. *lactis* strain AD011 is the strain deposited in GenBank under accession number CP001213.

An exemplary *Bifidobacterium animalis* subsp. *lactis* strain P23 is the strain deposited under accession number ATCC 27536.

An exemplary *Bifidobacterium animalis* subsp. *lactis* strain DN-173-010 is the strain deposited at CNCM under accession number 1-2494.

An exemplary *Bifidobacterium animalis* subsp. *lactis* strain CHCC3912 is the strain deposited under accession number DSM21406.

An exemplary *Bifidobacterium animalis* subsp. *lactis* strain CHCC5445, also known as BB-12®, is the strain deposited under accession number DSM15954 or as deposited in GenBank under accession number CP001853.

In certain embodiments, the dietary supplement as described herein may comprise at least 10⁶ colony-forming units (CFU), at least 10⁷ CFU, at least 10⁸ CFU, at least 10⁹ CFU, at least 10¹⁰ CFU, at least 10¹¹, at least 10¹², or at least 10¹³ CFU of the probiotic per 100 g of the dietary supplement.

In certain embodiments, the dietary supplement as described herein may comprise from 10⁶ to 10¹⁴ CFU, , from 10⁶ to 10¹² CFU or from 10⁷ to 10¹² CFU, or from 10⁸ to 10¹² CFU of said probiotic per 100 g of the dietary supplement. Preferably, the dietary supplement as described herein comprises from 10⁸ to 10¹⁴ CFU of said probiotic per 100 g of the dietary supplement. More preferably, the dietary supplement as described herein comprises from 10⁸ to 10¹² CFU of said probiotic per 100 g of the dietary supplement. For example, the dietary supplement as described herein comprises 1.22 x 10¹¹ CFU of said probiotic per 100 g of the dietary supplement.

In certain embodiments, a daily serving of the dietary supplement as taught herein may comprise at least 10⁶ CFU, at least 10⁷ CFU, at least 10⁸ CFU, at least 10⁹ CFU, at least 10¹⁰ CFU, at least 10¹¹ CFU, at least 10¹², or at least 10¹³ of said probiotic.

In certain embodiments, a daily serving of the dietary supplement as taught herein may comprise from 10⁶ to 10¹⁴ CFU, from 10⁸ to 10¹⁴ CFU, or from 10⁶ to 10¹² CFU of said probiotic. Preferably, a daily serving of the dietary supplement as described herein comprises from 10⁸ to 10¹² CFU of said probiotic. For example, a daily serving of the dietary supplement may comprise 10¹⁰ CFU of said probiotic.

The recitations "daily serving" and "daily dose" as used herein may be used interchangeably and refer to the recommended amount of dietary supplement as described herein to be consumed on a daily basis by a subject.

In certain embodiments, a daily serving of the dietary supplement may comprise from 3000 mg to 15000 mg of the dietary supplement. For instance, the daily serving of the dietary supplement may comprise from 4000 mg to 12500 mg, from 5000 mg to 12000 mg, from 6000 to 10000 mg, from 7000 mg to 9000 mg, from 7500 mg to 8500 mg, or from 8000 mg to 8500 mg of the dietary supplement. Preferably, the daily serving of the dietary supplement comprises from 6000 to 10000 mg of the dietary supplementFor example, the daily serving of the dietary supplement may comprise 8000 mg, 8100 mg, 8200 mg, 8300 mg, 8400 mg, or 8500 mg of the dietary supplement. In certain embodiments, the dietary supplement as described herein may comprise at least one ingredient selected from an ingredient capable of cleansing toxins and protecting against their build up, an ingredient capable of supporting lipid and fatty acid metabolism, an ingredient capable of supporting healthy digestion, wherein said ingredient capable of supporting healthy digestion is an ion or a salt thereof, and an ingredient capable of supporting healthy liver function.

The phrase "at least one ingredient" refers to one or more ingredients, such as two or more, three of more, or four or more, such as five, six, seven, eight or more ingredients.

The phrase "an ingredient capable of cleansing toxins and protecting against their build up" as used herein refers to any substance capable of cleansing toxins and protecting against their build up, such as a substance capable of supporting the functionality of organs such as the liver or kidneys; a substance having a draining effect; a substance capable of protecting against oxidative stress; a substance capable of cleansing gut; a substance capable of participating in the antioxidant defense system of the body; a substance capable of binding to thiol groups in proteins which may make them less susceptible to oxidation; a substance capable of displacing redox-reactive metal (e.g., iron or copper) from proteins or lipids which may reduce metal-induced formation of hydroxyl radicals; a substance capable of inducing the expression of scavengers of reactive oxygen species (e.g., metallothionein or catalase); and/or a cofactor of the antioxidant enzyme copper/zinc-superoxide.

The term "toxin" as used herein refers to any substance produced within living cells or organisms (e.g., bacteria in the gut) or any substance entering the body from the environment, and having a detrimental effect on cell function or structure. The term encompasses "endotoxins" and "exotoxins". "Endotoxins" (also known as lipopolysaccharides (LPS) or lipoglycans) are toxins typically found in the outer membrane of Gram-negative bacteria, for instance residing in the gut. "Exotoxins" are toxins excreted or secreted by organisms, such as by bacteria residing in the gut. Non-limiting examples of toxins are toxins produced by unwanted bacteria and yeast in the gut; industrial chemicals and their polluted by-products; pesticides; additives in our foods such as flavor enhancers, food colorings, preservatives and fillers; heavy metals; anesthetics; drug deposits; environmental hormones; secondary smoke; toxic byproducts from food metabolism; alcohol; solvents; formaldehyde; and herbicides.

The terms "detoxification", "cleansing" or "detox" refer to the physiological and/or medicinal removal of toxic substances from a subject. Non-limiting examples of benefits of detoxification include cleansing the digestive tract of accumulated waste, reducing food allergies, reducing unwanted organisms, blood purification, increased vitality, supporting weight loss, stronger immune system, improved skin, lowering blood pressure levels, reduce blood fat levels, improve liver organ function, reduced fluid retention and improved intestinal healthy.

The phrase "an ingredient capable of cleansing toxins and protecting against their build up" refers to one or more ingredients capable of cleansing toxins and protecting against their build up, such as two or more, three of more, or four or more, such as five, six, seven, eight or more ingredients capable of cleansing toxins and protecting against their build up.

The phrase "an ingredient capable of supporting lipid and fatty acid metabolism" as used herein may refer to any substance capable of supporting lipid ad fatty acid metabolism, such as a precursor of phospholipids; a substance capable of playing a role in lipid and/or cholesterol transport; a substance capable of playing a role in lipid and/or cholesterol metabolism; a substance required for the synthesis of prostaglandin belonging to series 1, for example by the conversion of linoleic acid to gamma-linolenic acid and/or the mobilization of dihomogammalinolenic acid.

The term "lipid" as used herein refers to a substance that is soluble in nonpolar solvents. Non-limiting examples of lipids are fatty acids, glycerolipids, glycerophospholipids, sphingolipids, sterol lipids, prenol lipids, saccharolipis and polyketides.

The term "fatty acid" as used herein refers to a carboxylic acid with an aliphatic chain comprising at least 4 carbon atoms (e.g., from 4 to 28 carbon atoms). The fatty acid may either comprise carbon-carbon double bonds, and accordingly may be referred to as "unsaturated fatty acid", such as monounsaturated fat, polyunsaturated fat and trans fat, including cholesterol. If the fatty acid does not comprise carbon-carbon double bonds, it may be referred to as a "saturated fatty acid". Fatty acids may be present in a subject's diet in the form of animal or vegetable fats and/or oils, such as lard, butter, milk fat, coconut oil, cocoa butter, palm kernel oil, soybean oil, corn oil, sunflower oil, hemp oil, rapeseed oil, wheat germ oil and cottonseed oil.

The phrase "an ingredient capable of supporting lipid and fatty acid metabolism" refers to one or more ingredients capable of supporting lipid and fatty acid metabolism, such as two or more, three of more, or four or more, such as five, six, seven, eight or more ingredients capable of supporting lipid and fatty acid metabolism.

The phrase "an ingredient capable of supporting healthy digestion" as used herein may refer to any substance capable of supporting healthy digestion, such as a substance capable of contributing to the production of hydrochloric acid in the stomach.

The term "healthy digestion" as used herein refers to a digestion by a functionally normal (e.g. regular uptake of nutrients, regular bowel transit time) and structurally normal (e.g. no blockages, punctures and/or malformations) gut. A healthy digestion is typically absent from gut issues such as bloating, diarrhea, inflammatory disorders and/or gut barrier dysfunction.

The phrase "an ingredient capable of supporting healthy digestion" refers to one or more ingredients capable of supporting healthy digestion, such as two or more, three of more, or four or more, such as five, six, seven, eight or more ingredients capable of supporting healthy digestion.

The phrase "an ingredient capable of supporting healthy liver function" as used herein may refer to any substance capable of supporting healthy liver function, such as a substance capable of the metabolism and transport of lipids and cholesterol or a substance capable of preventing the development of a fatty liver (hepatic steatosis, which can result in non-alcoholic fatty liver disease (NAFLD)). The term "healthy liver function" as used herein refers to the functioning of a structurally normal (e.g. no scarring, no malformations) and healthy (e.g. absence of any liver diseases such as fatty liver disease, cirrhosis, alcohol-related liver disease (ARLD), hepatitis, or cancer) liver. Non-limiting examples of liver functions include the regulation of the blood composition; the production of cholesterol, albumin and clotting factors; the breakdown of alcohol and medicaments; the detoxification of the blood; and the storage of nutrients such as iron and vitamin A.

The ingredient capable of cleansing toxins and protecting against their build up, the ingredient capable of supporting lipid and fatty acid metabolism, the ingredient capable of supporting healthy digestion, and the ingredient capable of supporting healthy liver function may also have beside their own capability the capabilities of one or more of the other ingredients. In other words, one ingredient may be capable of performing one or more functions, such as two, three or all four, of cleansing toxins and protecting against their build up, supporting lipid and fatty acid metabolism, supporting healthy digestion, and supporting healthy liver function.

The ingredient capable of cleansing toxins and protecting against their build up, the ingredient capable of supporting lipid and fatty acid metabolism, the ingredient capable of supporting healthy digestion, and the ingredient capable of supporting healthy liver function may be vitamins, vitamin-like substances, minerals, anti-oxidants, or herbal substances or compositions.

The term "vitamin" as used herein refers to an organic compound which is essential for normal growth and/or nutrition of a subject, such as a human. Vitamins are typically required in small quantities in the diet of the subject or as a supplement to the diet. Non-limiting examples of vitamins include vitamin A, vitamin B1, vitamin B2, vitamin B3, vitamin B5, vitamin B6, vitamin B7, vitamin B9, vitamin B12, vitamin C, vitamin D, vitamin E and vitamin K. The term "vitamin-like substances" as used herein refer to organic compounds, related to vitamins in activity, which are synthesized by an organism in adequate amounts and are therefore not required in the diet. Non-limiting examples of vitamin-like nutrients are choline, myo-inositol, para-aminobenzoic acid, carnitine, lipoic acid and bioflavonoids.

The term "mineral" as used herein refers to an edible metal or a salt thereof such as zinc or a salt thereof, selenium or a salt thereof, tin or a salt thereof, and copper or a salt thereof.

The term "anti-oxidant" as used herein refers to a substance capable of neutralizing harmful free radicals. Non-limiting examples of anti-oxidants are vitamin C, vitamin E, resveratrol and citric acid.

The term "herbal substance or composition" may refer to a substance or composition obtained from plants, plant parts (e.g., roots, seeds, leaves, flowers), or extracts of plants or parts thereof.

In certain embodiments, the ingredient capable of cleansing toxins and protecting against their build up may be selected from choline or a salt thereof, vitamin B or a salt thereof, zinc or a salt thereof, vitamin C or a salt thereof, selenium or a salt thereof, and vitamin E or a salt thereof. Preferably, the ingredient capable of cleansing toxins and protecting against their build up is selected from choline or a salt thereof, vitamin B or a salt thereof, and zinc or a salt thereof.

In certain embodiments, the ingredient capable of supporting lipid and fatty acid metabolism may be selected from zinc or a salt thereof, and choline or a salt thereof.

In certain embodiments, the ingredient capable of supporting healthy digestion is an ion or a salt thereof.

In certain embodiments, the ingredient capable of supporting healthy digestion may be selected from chloride or a salt thereof, and calcium or a salt thereof. Preferably, the ingredient capable of supporting healthy digestion is chloride or a salt thereof.

In certain embodiments, the salt capable of supporting healthy digestion is a chloride salt.

In certain embodiments, the ingredient capable of supporting healthy liver function may be choline or a salt thereof. In certain embodiments, the ingredient capable of cleansing toxins and protecting against their build up is selected from choline or a salt thereof, vitamin B or a salt thereof, and zinc or a salt thereof; the ingredient capable of supporting lipid and fatty acid metabolism is selected from zinc or a salt thereof, and choline or a salt thereof; the ingredient capable of supporting healthy digestion is chloride or a salt thereof; and the ingredient capable of supporting healthy liver function is choline or a salt thereof.

The term "chloride" or "Cl⁻" as used herein refers to the negatively charged ion from Chlorine which can be referred to by its CAS registry number, namely 16887-00-6. Chloride may be present in the form of an ion or a chloride salt. Chloride may act as an essential mineral for subjects.

In certain embodiments, the chloride salt may be selected from the group consisting of potassium chloride, sodium chloride, magnesium chloride, and ammonium chloride. Preferably, the chloride salt is potassium chloride.

In certain embodiments, the dietary supplement as described herein may comprise from 0.01 to 35 percent by weight of chloride or the salt thereof. For instance, the dietary supplement as described herein may comprise from 0.01 to 20 percent by weight, from 0.01 to 10 percent by weight, from 0.01 to 5 percent by weight, from 0.01 to 2 percent by weight, from 0.05 to 35 percent by weight, from 0.1 to 35 percent by weight, from 0.5 to 35 percent by weight, from 1 to 35 percent by weight, from 0.5 to 20 percent by weight, from 0.5 to 10 percent by weight, from 0.5 to 5 percent by weight, from 0.5 to 2 percent by weight, from 1 to 20 percent by weight, from 1 to 10 percent by weight, from 1 to 5 percent by weight, or from 1 to 2 percent by weight of chloride or the salt thereof. Preferably, the dietary supplement as described herein comprises from 0.9 to 2.2 percent by weight of chloride or the salt thereof. For example, the dietary supplement as described herein may comprise from 0.97 to 2.12 percent by weight of chloride or the salt thereof, such as 1.5 percent by weight of chloride or the salt thereof.

In certain embodiments, a daily serving of the dietary supplement as taught herein may comprise from 1 mg to 3000 mg of chloride or the salt thereof. For instance, the daily serving of the dietary supplement as described herein may comprise from 1 mg to 2000 mg, from 1 mg to 1000 mg, from 1 mg to 500 mg, from 1 mg to 200 mg, from 1 mg to 140 mg, from 10 mg to 3000 mg, from 50 mg to 3000 mg, from 100 mg to 3000 mg, from 10 mg to 1000 mg, from 50 mg to 1000 mg, from 100 mg to 1000 mg, from 10 mg to 500 mg, from 50 mg to 500 mg, from 100 mg to 500 mg, from 10 mg to 200 mg, from 50 mg to 200 mg, from 100 mg to 200 mg, or from 100 mg to 140 mg of chloride or the salt thereof. Preferably, the daily serving of the dietary supplement as described herein comprises from 75 mg to 175 mg of chloride or the salt thereof. For example, the daily serving of the dietary supplement as taught herein may comprise from 79.54 mg to 173.84 mg of chloride or the salt thereof, such as 120 mg of chloride or the salt thereof.

The term "choline" as used herein refers to a vitamin-like essential nutrient of the class of quaternary ammonium salts containing the N,N,N-trimethylethanolammonium cation. Choline typically is a quaternary ammonium salt with chemical formula (CH₃)₃N+(CH₂)₂OHX-, where X⁻ is a counterion such as chloride, hydroxide or tartrate. Choline can also be referred to by its CAS registry number, namely 62-49-7, or by its IUPAC name, namely 2-hydroxy-N,N,N-trimethylethan-1-aminium.

In certain embodiments, the choline salt may be choline chloride, choline bitartrate, citicoline (CDP-choline), L-alpha-glycerophosphocholine (Alpha-GPC) and phosphatidylcholine, preferably the choline salt is choline bitartrate.

In certain embodiments, the dietary supplement as described herein may comprise from 0.01 to 6 percent by weight of choline or the salt thereof. For instance, the dietary supplement as described herein may comprise from 0.01 to 3 percent by weight, from 0.01 to 2 percent by weight, from 0.01 to 1.5 percent by weight, from 0.05 to 6 percent by weight, from 0.1 to 6 percent by weight, from 0.25 to 6 percent by weight, from 0.5 to 6 percent by weight, from 0.1 to 3 percent by weight, from 0.1 to 2 percent by weight, from 0.25 to 3 percent by weight, from 0.25 to 2 percent by weight, or from 0.5 to 1.5 percent by weight of choline or the salt thereof. Preferably, the dietary supplement as described herein comprises from 0.8 to 1.5 percent by weight of choline or the salt thereof. For example, the dietary supplement as described herein may comprise 1 percent by weight of choline or the salt thereof.

In certain embodiments, a daily serving of the dietary supplement as taught herein may comprise from 1 mg to 500 mg of choline or the salt thereof. For instance, the daily serving of the dietary supplement as described herein may comprise from 1 mg to 400 mg, from 1 mg to 300 mg, from 1 mg to 200 mg, from 1 mg to 100 mg, from 10 mg to 500 mg, from 25 mg to 500 mg, from 50 mg to 500 mg, from 65 mg to 500 mg, from 10 mg to 200 mg, from 25 mg to 200 mg, from 50 mg to 200 mg, from 50 mg to 100 mg, from 1 mg to 500 mg, or from 65 mg to 100 mg of choline or the salt thereof. Preferably, the daily serving of the dietary supplement as described herein comprises from 65mg to 125 mg of choline or the salt thereof. For example, the daily serving of the dietary supplement as taught herein may comprise from 65.6 mg to 123 mg of choline or the salt thereof, such as 82.5 mg of choline or the salt thereof.

The term "zinc" or "Zn" as used herein refers to an essential mineral with atomic number 30, which can be categorized in the periodic table under the group of volatile metals (i.e. group 12). Zinc can be also be referred to by its CAS registry number, namely 7440-66-6.

In certain embodiments, the zinc salt may be a zinc salt selected from the group consisting of zinc gluconate, zinc acetate, zinc picolinate, zinc amino acid chelate, zinc chloride, zinc citrate, zinc bisglycinate chelate, zinc stearate, zinc lactate, zinc ammonium sulfate, zinc chromate, zinc dithionate, zinc fluorosilicate, zinc tartrate, zinc formate, zinc iodide, zinc nitrate, zinc phenol sulfonate, zinc salicylate, zinc sulfate, zinc succinate, zinc glycerophosphate, and zinc halides. Preferably, the zinc salt is zinc gluconate.

In certain embodiments, the dietary supplement as described herein may comprise from 0.001 to 0.25 percent by weight of zinc or the salt thereof. For instance, the dietary supplement as described herein may comprise from 0.001 to 0.20 percent by weight, from 0.001 to 0.1 percent by weight, from 0.001 to 0.05 percent by weight, from 0.001 to 0.04 percent by weight, from 0.005 to 0.25 percent by weight, from 0.005 to 0.20 percent by weight, from 0.005 to 0.1 percent by weight, from 0.005 to 0.05 percent by weight, from 0.005 to 0.04 percent by weight, from 0.01 to 0.25 percent by weight, from 0.01 to 0.20 percent by weight, from 0.01 to 0.1 percent by weight, from 0.01 to 0.05 percent by weight, from 0.01 to 0.04 percent by weight, or from 0.01 to 0.03 percent by weight of zinc or the salt thereof. Preferably, the dietary supplement as described herein comprises from 0.01 to 0.02 percent by weight of zinc or the salt thereof. For example, the dietary supplement as described herein may comprise 0.02 percent by weight of zinc or the salt thereof.

In certain embodiments, a daily serving of the dietary supplement as taught herein may comprise from 0.08 mg to 20 mg of zinc or the salt thereof. For instance, the daily serving of the dietary supplement as described herein may comprise from 0.1 mg to 15 mg, from 0.1 mg to 10 mg, from 0.1 mg to 5 mg, from 0.1 mg to 4 mg, from 0.1 mg to 3 mg, from 0.1 mg to 2 mg, from 0.5 mg to 20 mg, from 0.5 mg to 15 mg, from 0.5 mg to 10 mg, from 0.5 mg to 5 mg, from 0.5 mg to 4 mg, from 0.5 mg to 3 mg, from 0.5 mg to 2 mg, from 1 mg to 20 mg, from 1 mg to 15 mg, from 1 mg to 10 mg, from 1 mg to 5 mg, from 1 mg to 4 mg, from 1 mg to 3 mg, or from 1 mg to 2 mg of zinc or the salt thereof. Preferably, the daily serving of the dietary supplement as described herein comprises from 0.8 mg to 1.7 mg of zinc or the salt thereof. For example, the daily serving of the dietary supplement as taught herein may comprise from 0.82 mg to 1.64 mg of zinc or the salt thereof, such as 1.5 mg of zinc or the salt thereof.

The term "vitamin B2", "vitB2", "riboflavin", "vactochrome", "lactoflavin" or "vitamin G", as used herein may be used interchangeably and refers to a vitamin represented by chemical formula C₁₇H₂₀N₄O₆. Vitamin B2 can also be referred to by its CAS registry number, namely 83-88-5, or by its IUPAC name, namely 2-hydroxy-N,N,N-trimethylethan-1-aminium.

In certain embodiments, the dietary supplement as described herein may comprise from 0.0005 to 0.02 percent by weight of vitamin B2. For instance, the dietary supplement as described herein may comprise from 0.001 to 0.01 percent by weight, from 0.001 to 0.005 percent by weight, from 0.001 to 0.004 percent by weight, from 0.001 to 0.0035 percent by weight, from 0.0015 to 0.02 percent by weight, from 0.0015 to 0.01 percent by weight, from 0.0015 to 0.005 percent by weight, from 0.0015 to 0.004 percent by weight, or from 0.0015 to 0.0035 percent by weight of vitamin B2. Preferably, the dietary supplement as described herein comprises from 0.002 to 0.004 percent by weight of vitamin B2. For example, the dietary supplement as described herein may comprise 0.003 percent by weight of vitamin B2.

In certain embodiments, a daily serving of the dietary supplement as taught herein may comprise from 0.04 mg to 2 mg of vitamin B2. For instance, the daily serving of the dietary supplement as described herein may comprise from 0.1 mg to 1 mg, from 0.1 mg to 0.5 mg, from 0.1 mg to 0.4 mg, from 0.1 mg to 0.3 mg, from 0.15 mg to 2 mg, from 0.15 mg to 1 mg, from 0.15 mg to 0.5 mg, from 0.15 mg to 0.4 mg, or from 0.1 to 0.4 mg of vitamin B2. Preferably, the daily serving of the dietary supplement as described herein comprises from 0.1 mg to 0.4 mg of vitamin B2. For example, the daily serving of the dietary supplement as taught herein may comprise from 0.164 mg to 0.328 mg of vitamin B2, such as 0.210 mg of vitamin B2.

In certain embodiments, the dietary supplement as described herein may further comprise a prebiotic.

The citation "a prebiotic" includes both singular and plural prebiotics unless the context clearly dictates otherwise. The term "prebiotic" as used herein, refers to non-digestible food ingredients which beneficially affect the health of the host when consumed. Such health benefits may include, but are not limited to, selective stimulation of the growth and/or activity of one or a limited number of beneficial gut bacteria, stimulation of the growth and/or activity of ingested probiotics, improved gut health, improved digestion, enhanced immune function, reduced risk for heart diseases, hormone regulation, improved bone health and increased resistance to invading pathogens. Such prebiotics may be naturally-occurring, synthetic, or developed through the genetic manipulation of organisms and/or plants.

In certain embodiments, the prebiotic may be a food fiber. The prebiotic may be a soluble food fiber (e.g., soluble in water) or an insoluble food fiber (e.g., insoluble in water).

In certain embodiments, the prebiotic is a soluble food fiber.

In certain embodiments, the prebiotic may be one or more soluble food fibers selected from the group consisting of maltodextrin, inulin, oligofructose, lactose, lactulose, resistant starch, fructooligosaccharides (FOS), galactooligosaccharides (GOS), transgalactooligosaccharides (TOS), wheat bran-derived arabinoxylooligosaccharides (AXOS), xylooligosaccharides (XOS), isomaltooligosaccharides (IMO), gentiooligosaccharides (GTO), glucooligosaccharides, pectic oligosaccharides, soy oligosaccharides, lactosucrose, polydextrose, sugar alcohols, alpha glucan, beta glucan, wheat dextrin, guar gum, flaxseed gum, fenugreek gum, acacia gum, psyllium, and raffinose. Preferably, the prebiotic is maltodextrin.

In certain embodiments, the dietary supplement as described herein comprises from 1 to 95 percent by weight of said prebiotic. For instance, the dietary supplement as described herein may comprise from 10 to 95 percent by weight, from 20 to 95 percent by weight, from 30 to 95 percent by weight, from 40 to 95 percent by weight, from 50 to 95 percent by weight, from 60 to 95 percent by weight, from 65 to 95 percent by weight, from 65 to 90 percent by weight, or from 65 to 85 percent by weight of said prebiotic. Preferably, the dietary supplement as described herein comprises from 55 to 90 percent by weight of said prebiotic. For example, the dietary supplement as described herein may comprise from 58 to 88 percent by weight of said prebiotic, such as 74 percent by weight of said prebiotic.

In certain embodiments, a daily serving of said dietary supplement as described herein comprises from 100 mg to 15000 mg of said prebiotic. For instance, the daily serving of the dietary supplement as described herein may comprise from 500 mg to 15000 mg, from 500 mg to 10000 mg, from 1000 mg to 10000 mg, from 1000 mg to 8000 mg, from 2500 mg to 8000 mg, from 5000 mg to 8000 mg, or from 5000 mg to 7000 mg of said prebiotic. Preferably, the daily serving of the dietary supplement as described herein comprises from 4758 mg to 7216 mg of said prebiotic. For example, the daily serving of said dietary supplement as described herein may comprise 6030 mg of said prebiotic.

In certain embodiments, the dietary supplement as described herein may comprise a probiotic; chloride or a salt thereof; choline or a salt thereof; zinc or a salt thereof; vitamin B2; and a prebiotic.

In certain embodiments, the dietary supplement as described herein may comprise a probiotic; a chloride salt; choline or a salt thereof; zinc or a salt thereof; vitamin B2; and a prebiotic.

In certain embodiments, the dietary supplement as described herein may comprise:
- from 10⁶ to 10¹⁴ CFU, preferably from 10⁸ to 10¹² CFU, of the probiotic per 100 g of said dietary supplement;
- from 0.01 to 35 percent by weight, preferably from 0.9 to 2.2percent by weight, of chloride or the salt thereof;
- from 0.01 to 6 percent by weight, preferably from 0.8to 1.5 percent by weight, of choline or the salt thereof;
- from 0.001 to 0.25 percent by weight, preferably from 0.01 to 0.02 percent by weight, of zinc or the salt thereof;
- from 0.0005 to 0.02 percent by weight, preferably from 0.002 to 0.004 percent by weight, of vitamin B2; and/or
- from 1 to 95 percent by weight, preferably from 55 to 90 percent by weight, of said prebiotic.

A dietary supplement may comprise for example the following ingredients, typically in the following amounts (in % by weight of the dietary supplement):
- from 10⁸ to 10¹² CFU of the probiotic per 100 g of said dietary supplement;
- from 0.97 to 2.12 percent by weight of chloride or the salt thereof;
- from 0.8 to 1.5 percent by weight of choline or the salt thereof;
- from 0.01 to 0.02 percent by weight of zinc or the salt thereof;
- from 0.002 to 0.004 percent by weight of vitamin B2; and/or
- from 58 to 88 percent by weight of said prebiotic.

In certain embodiments, a daily serving of said dietary supplement may comprise:
- from 10⁶ to 10¹⁴ CFU, preferably from 10⁸ to 10¹² CFU, of said probiotic;
- from 1 mg to 3000 mg, preferably from 75 mg to 175 mg, of chloride or the salt thereof;
- from 1 mg to 500 mg, preferably from 65mg to 125 mg, of choline or the salt thereof;
- from 0.08 mg to 20 mg, preferably from 0.8 mg to 1.7 mg, of zinc or the salt thereof;
- from 0.04 mg to 2 mg, preferably from 0.1 mg to 0.4 mg, of vitamin B2; and/or
- from 100 mg to 15000 mg, preferably 4500 mg to 7500 mg, of said prebiotic.

A dietary supplement may comprise for example the following ingredients, typically in the following amounts (in % by weight of the dietary supplement):- from 10⁸ to 10¹² CFU of said probiotic;
- from 79.54 mg to 173.84 mg of chloride or the salt thereof;
- from 65.6 mg to 123 mg of choline or the salt thereof;
- from 0.82 mg to 1.64 mg of zinc or the salt thereof;
- from 0.164 mg to 0.328 mg of vitamin B2; and/or
- from 4758 mg to 7216 mg of said prebiotic.

In certain embodiments, the probiotic, the chloride or salt thereof, the choline or salt thereof, the zinc or salt thereof, vitamin B2, and optionally the prebiotic, may be the only active ingredients present in the dietary supplement as described herein. Hence, certain embodiments provide a dietary supplement consisting of the probiotic, the chloride or salt thereof, the choline or salt thereof, the zinc or salt thereof, vitamin B2, and optionally the prebiotic.

The terms "active ingredient" or "active component" can be used interchangeably and broadly refer to a compound or substance which, when provided in an effective amount, achieves a desired therapeutic and/or prophylactic outcome(s). Typically, an active ingredient may achieve such outcome(s) through interacting with and/or modulating living cells or organisms. The term "active" in the recitations "active ingredient" or "active component" refers to "pharmacologically active" and/or "physically active".

In certain embodiments, the dietary supplement as described herein may be stable for at least 2 months, at least 3 months, at least 6 months, at least 12 months, at least 18 months, at least 24 months, at least 30 months, at least 36 months, or at least 42 months. Preferably, the dietary supplement as described herein may be stable for at least 24 months.

In certain embodiments, the dietary supplement as described herein may be stable for at least 2 months, at least 3 months, at least 6 months, at least 12 months, at least 18 months, at least 24 months, at least 30 months, at least 36 months, at least 42 months, preferably at least 24 months, when stored at a temperature from 0°C to 4°C (i.e. refrigerated or cooled), preferably a temperature of 4°C. Preferably, the dietary supplement as described herein may be stable for at least 24 months when stored at a temperature of 4°C.

In certain embodiments, the dietary supplement as described herein may be stable for at least 2 months, at least 3 months, at least 6 months, at least 12 months, at least 18 months, at least 24 months, at least 30 months, at least 36 months, at least 42 months, preferably at least 24 months, when stored at ambient temperature (i.e. room temperature). In certain embodiments, the dietary supplement as described herein may be stable for at least 2 months, at least 3 months, at least 6 months, at least 12 months, at least 18 months, at least 24 months, at least 30 months, at least 36 months, at least 42 months, preferably at least 24 months, when stored at a temperature from 10°C to 25°C, from 15°C to 25°C, from 18°C to 25°C, or from 18°C to 22°C. Preferably, the dietary supplement as described herein may be stable for at least 24 months when stored at a temperature from 10°C to 25°C.The term "stability" as used herein refers to resistance to chemical change, disintegration, loss of activity, loss of efficacy and/or loss of quality of the dietary supplement as described herein or of one or more ingredients thereof over time under storage or use. The stability of a dietary supplement during storage may also be referred to as the "shelf-life" of said dietary supplement. Stability of the dietary supplement or of one or more ingredients thereof may be determined by any methods known in the art to determine the stability of a dietary supplement or ingredient thereof. For example, the stability of the dietary supplement or of one or more ingredients thereof may be determined by the evaluation of parameters such as organoleptic properties (e.g. color, flavor, odor, and texture), dietary ingredient strengths, chemical fingerprints, ingredient degradation products, microbial growth, preservative content, moisture content, disintegration, dissolution, pH, viscosity and oxidation. Environmental conditions that may lead to instability of the dietary supplement or of one or more ingredient of the dietary supplement include humidity, shock and temperature.

In certain embodiments, the dietary supplement as described herein may retain at least 1.22 x 10⁹ CFU of the probiotic per gram of said dietary supplement, when being stored for at least 2 months, at least 3 months, at least 6 months, at least 12 months, at least 18 months, at least 24 months, at least 30 months, at least 36 months, at least 42 months, preferably at least 24 months.

In certain embodiments, the dietary supplement as described herein may retain at least 1.22 x 10⁹ CFU of the probiotic per gram of said dietary supplement when being stored for at least 2 months, at least 3 months, at least 6 months, at least 12 months, at least 18 months, at least 24 months, at least 30 months, at least 36 months, at least 42 months, preferably at least 24 months at 4°C degrees.

In certain embodiments, the dietary supplement as described herein may retain at least 1.22 x 10⁹ CFU of the probiotic per gram of said dietary supplement when being stored for at least 2 months, at least 3 months, at least 6 months, at least 12 months, at least 18 months, at least 24 months, at least 30 months, at least 36 months, at least 42 months, preferably at least 24 months at ambient temperature (i.e. room temperature). In certain embodiments, the dietary supplement as described herein may retain at least 1.22 x 10⁹ CFU of the probiotic per gram of said dietary supplement when being stored for at least 2 months, at least 3 months, at least 6 months, at least 12 months, at least 18 months, at least 24 months, at least 30 months, at least 36 months, at least 42 months, preferably at least 24 months at a temperature from 10°C to 25°C, from 15°C to 25°C, or from 18°C to 22°C. Preferably, the dietary supplement as described herein may retain at least 1.22 x 10⁹ CFU of the probiotic per gram of said dietary supplement when being stored for at least 24 months at a temperature from 10°C to 25°C.In certain embodiments, a daily serving of the dietary supplement as described herein may retain at least 1 x 10¹⁰ CFU of the probiotic when being stored for at least 2 months, at least 3 months, at least 6 months, at least 12 months, at least 18 months, at least 24 months, at least 30 months, at least 36 months, at least 42 months, preferably at least 24 months.

In certain embodiments, a daily serving of the dietary supplement as described herein may retain at least 1 x 10¹⁰ CFU of the probiotic when being stored for at least 2 months, at least 3 months, at least 6 months, at least 12 months, at least 18 months, at least 24 months, at least 30 months, at least 36 months, at least 42 months, preferably at least 24 months at 4°C degrees.

In certain embodiments, a daily serving of the dietary supplement as described herein may retain at least 1 x 10¹⁰ CFU of the probiotic when being stored for at least 2 months, at least 3 months, at least 6 months, at least 12 months, at least 18 months, at least 24 months, at least 30 months, at least 36 months, at least 42 months, preferably at least 24 months at ambient temperature (i.e. room temperature). In certain embodiments, a daily serving of the dietary supplement as described herein may retain at least 1 x 10¹⁰ CFU of the probiotic when being stored for at least 2 months, at least 3 months, at least 6 months, at least 12 months, at least 18 months, at least 24 months, at least 30 months, at least 36 months, at least 42 months, preferably at least 24 months at a temperature from 10°C to 25°C, from 15°C to 25°C, or from 18°C to 22°C. Preferably, a daily serving of the dietary supplement as described herein may retain at least 1 x 10¹⁰ CFU of the probiotic when being stored for at least 24 months at a temperature from 10°C to 25°C.

In certain embodiments, a daily dose of the dietary supplement may be properly (i.e. for at least 95%) solubilized in a defined amount of water, preferably wherein the defined amount of water is from 150 ml to 300 ml of water, more preferably wherein the defined amount of water is from 200 ml to 250 ml of water.

In certain embodiments, the dietary supplement may comprise one or more components selected from the group consisting of flavouring agents, colouring agents, acidifying agents, and anticaking agents .

The term "flavouring agent", "flavour substance", "flavour extract" or "flavour preparation" as used herein refers to food-grade natural or chemical substances which are used as additive to a preparation to create, enhance or modify taste and/or the aroma of the preparation. Flavouring agents may have a sweet, sour, salty, bitter and/or umami taste. Non-limiting examples of food-grade flavouring agents include substances with E number E620, E621, E622, E623, E624, E625, E626, E627, E628, E629, E630, E631, E632, E633, E634, E635, E640 and E650.

In certain embodiments, the flavouring agent may be a sweetening, an acidulant, or a bitter flavouring. An example of the sweetening may be aspartame, stevia, saccharin sodium, or thaumatin. An example of the acidulant may be citric acid, malic acid, tartaric acid, or fumaric acid. An example of the bitter flavouring may be caffeine or naringin.

In certain embodiments, the flavouring agent is a citrus flavour, such as a lemon flavour, an orange flavour, a grapefruit flavour or a lime flavour, preferably the flavouring agent is a lemon flavour.

The term "colouring agent" as used herein refers to a food-grade dye, pigment or substance that imparts colour when added to the dietary supplement. The colouring agent may be in the form of a liquid, a powder, a gel or a paste. Non-limiting examples of food-grade colouring agents include substances with E number E100, E100, E101, E102, E103, E104, E105, E106, E107, E110, E120, E122, E123, E124, E127, E128, E129, E131, E132, E133, E140, E141, E142, E150a, E150b, E150c, E150d, E151, E153, E154, E155, E160a, E160b, E160c, E160d, E160e, E160f, E161a, E161b, E161c, E161d, E161h, E161i, E162, E163, E180 and E181.

The term "acidifying agent" as used herein refers to food-grade natural or chemical substances which are used as an additive to a preparation in order to create, enhance or modify the acidity of a preparation. Non-limiting examples of food-grade acidifying agents include vinegar, lactic acid (E270), acetic acid (E260), orthophosphoric acid (E338), citric acid (E 330) calcium acetate (E 263) and fumaric acid (E 297).

In certain embodiments, the acidifying agent is citric acid.

The term "anticaking agent" as used herein refers to a food-grade additive to prevent the formation of lumps in powdered or granulated material to make packaging, transport and/or consumption thereof easier. Non-limiting examples of food-grade anticaking agents include substances with E number E343, E421, E470b, E470a, E504, E530, E535, E536, E537, E538, E542, E550, E551, E552, E553a, E553b, E554, E555, E556, E557, E558, E559, E560, E570 and E572.

In certain embodiments, the anticaking agent is silicon dioxide (E551).

As indicated above, the dietary supplement as described herein may be a partial nutritional composition, which does not contain all the essential the essential macro- and micronutrients, and hence may not be used to replace one or more daily meals and/or as the sole source of nutrition, but can be taken in supplement to daily meals.

Accordingly, in certain embodiments, the dietary supplement as described herein comprises no fatty acids in addition to fatty acids of the prebiotic and/or probiotic (e.g., fatty acids endogenously present in the probiotic).

In certain embodiments, the dietary supplement as described herein has a total fatty acid content of at most 1 g, at most 0.5 g, at most 0.25 g, or at most 0.1 g per 100 kcal. Preferably, the dietary supplement as described herein has a total fatty acid content of at most 1 g per 100 kcal.

In certain embodiments, the dietary supplement as described herein comprises no carbohydrates in addition to the carbohydrates of the prebiotic and/or probiotic (e.g., carbohydrates of the prebiotic).

The term "carbohydrate" or "saccharide" as used herein refers to a biological molecule comprising carbon, hydrogen and oxygen. Non-limiting examples of carbohydrates are monosaccharides, such as fructose, glucose and galactose, disaccharides such as sucrose, lactose and maltose, oligosaccharides such as maltodextrin, raffinose and stachyose, and polysaccharides, such as starch, glycogen and cellulose.

In certain embodiments, the dietary supplement as described herein may comprise at most 0.5 g, at most 1 g, at most 2 g, at most 3 g, at most 4 g, or at most 5 g of carbohydrates per 100 kcal. Preferably, the dietary supplement as described herein comprises at most 0.5 g per 100 kcal.

In certain embodiments, the dietary supplement as described herein does not comprise vitamins and/or vitamin-like compounds in addition to vitamin B2, choline, vitamins, and vitamin-like compounds comprised within (e.g., endogenously present in) and/or produced by the probiotic.

In certain embodiments, the dietary supplement as described herein does not comprise minerals in addition to chloride, zinc, and minerals comprised within and/or produced by the probiotic.

In certain embodiments, the dietary supplement as described herein does not comprise any fatty acids and/or carbohydrates in addition to fatty acids and/or carbohydrates comprised within the prebiotic and/or probiotic.

In certain embodiments, the dietary supplement as described herein does not comprise any fatty acids, carbohydrates, vitamins, vitamin-like compounds, and/or minerals in addition to vitamin B2, chloride, choline, zinc, fatty acids, carbohydrates, vitamins, vitamin-like compounds, and/or minerals comprised within or produced by the prebiotic and/or probiotic.

In certain embodiments, the dietary supplement as described herein may be configured for oral administration.

The recitation "configured for oral administration" as used herein refers to the capability of the dietary supplement to be administered through the oral cavity of a subject to the digestive system. Oral delivery of the dietary supplement allows the ingredients of the dietary supplements to enter the gastrointestinal tract of the subject.

In certain embodiments, the dietary supplement as described herein may be in the form of a powder, a swallowable tablet, a sublingual tablet, an effervescent tablet, a chewable tablet, a capsule, a pastille, or a liquid preparation, preferably a powder.

The term "tablet" as used herein refers to a solid dosage form of one or more substances which may be prepared by moulding and/or by compression of a powder comprising said one or more substances. The tablet may comprise excipients such as diluents, binders or granulating agents, glidants and lubricants to ensure efficient tabletting; disintegrants to promote tablet break-up in the digestive tract; sweeteners or flavours to enhance taste; and/or pigments to make the tablets visually attractive or aid in visual identification of an unknown tablet. A polymer coating may be applied to the tablet to make the tablet smoother and easier to swallow, to control the release rate of the active ingredient, to increase its stability, and/or to enhance its appearance. Tablets may be specifically configured to dissolve sublingual (i.e. under the tongue), to be effervescent, to be chewable and/or to be swallowable.

The term "capsule" as used herein refers to a dosage form in which a solid covering material encapsulates one or more substances. The covering material may be composed of starches, degraded or chemically or physically modified starches (in particular dextrins and maltodextrins), gelatins, gum arabic, agar-agar, ghatti gum, gellan gum, modified and non-modified celluloses, pullulan, curdlan, carrageenans, alginic acid, alginates, pectin, inulin, xanthan gum and mixtures of two or more of said substances. The covering may dissolve in the mouth or stomach or may burst upon chewing to release the one or more substances comprised therein.

The term "pastille" as used herein refers to a dosage form of one or more substances, which is meant to be consumed by chewing and/or sucking, allowing it to dissolve in the mouth and release the substance or composition.

In certain embodiments, the dietary supplement as described herein may further comprise one or more pharmaceutical excipients. Suitable pharmaceutical excipients depend on the dosage form and identities of the active ingredients and can be selected by the skilled person (e.g. by reference to the Handbook of Pharmaceutical Excipients 6th Edition 2009, eds. Rowe et al.). As used herein, "carrier" or "excipient" includes any and all solvents, diluents, buffers (such as neutral buffered saline or phosphate buffered saline), solubilisers, colloids, dispersion media, vehicles, fillers, chelating agents (such as EDTA or glutathione), amino acids (such as glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the active ingredients.

A further aspect provides a dietary supplement comprising, consisting essentially of or consisting of: a probiotic; a prebiotic; chloride or a salt thereof; choline or a salt thereof; zinc or a salt thereof; and vitamin B2.

The skilled person will understand that the particular embodiments of the dietary supplement for weight loss and/or weight maintenance, wherein the dietary supplement comprises: at least one probiotic and at least one ingredient selected from: an ingredient capable of cleansing toxins and protecting against their build up, an ingredient capable of supporting lipid and fatty acid metabolism, an ingredient capable of supporting healthy digestion, and an ingredient capable of supporting healthy liver function as described herein also apply for the dietary supplement comprising, consisting essentially of or consisting of a probiotic; a prebiotic; chloride or a salt thereof; choline or a salt thereof; zinc or a salt thereof; vitamin B2; and a prebiotic.

Another aspect provides a sachet comprising the dietary supplement as described herein. Advantageously, the present sachet allows the subject to consume a daily dose of the dietary supplement in a straightforward manner. The present sachet can be taken along without difficulty and allows its content to be easily dissolved in water or another liquid prior to consumption.

The term "sachet" as used herein refers to a small bag, packet, case, pouch or pad suitable for containing a powder or liquid. The sachet may be made out of plastic, paper, metal (e.g. metal foil, such as aluminium foil) or a combination thereof. The sachet typically provides a barrier to external oxygen and/or moisture.

In certain embodiments, the sachet comprising the dietary supplement as described herein is closed airtight (i.e. hermetically).

In certain embodiments, the sachet comprising the dietary supplement as described herein has a tear-off top and/or a zip closure.

In certain embodiments, the sachet comprising the dietary supplement as described herein may have a volume of between 10 cm³ and 25 cm³, or between 10 cm³ and 20 cm³. Preferably, the sachet comprising the dietary supplement as described herein has a volume of between 15 cm³ and 20 cm³.

In certain embodiments, the sachet comprising the dietary supplement as described herein may have a height of between 6 cm and 16 cm, or between 8 cm and 14 cm. Preferably, the sachet comprising the dietary supplement as described herein has a height of between 10 cm and 12 cm..

In certain embodiments, the sachet comprising the dietary supplement as described herein may have a width of between 1 cm and 5 cm, or between 2 cm and 4 cm. For example, the sachet comprising the dietary supplement as described herein has a width of 3.5 cm.

In certain embodiments, the sachet comprises a single-serving quantity of the dietary supplement as described herein. In certain embodiments, the sachet comprises a daily serving quantity of the dietary supplement as described herein.

In certain embodiments, the sachet comprises the dietary supplement as described herein in the form of a powder.

In certain embodiments, the sachet may comprise from 3000 mg to 15000 mg of the dietary supplement. For instance, the sachet may comprise from 4000 mg to 12500 mg, from 5000 mg to 12000 mg, from 6000 to 10000 mg, from 7000 mg to 9000 mg, from 7500 mg to 8500 mg, or from 8000 mg to 8500 mg of the dietary supplement. Preferably, the sachet comprises from 6000 to 10000 mg of the dietary supplement. For example, the sachet may comprise 8000 mg, 8100 mg, 8200 mg, 8300 mg, 8400 mg, or 8500 mg of the dietary supplement.

Advantageously, the dietary supplement as taught herein allows to help a subject to prepare its body in advance of a weight loss program. The dietary supplement embodying the principles of the present invention helps to cleanse toxins from the digestive system and protects against further build-up of toxins, thereby allowing the subject to "detox" or "cleanse" its body in preparation of a weight loss treatment. Further, the present dietary supplement allows to support a subject's healthy fat metabolism and to maintain healthy digestion by contributing to gut flora balance. Thereby, the present dietary supplement may improve the results of a follow-up weight loss program conducted by the subject.

Accordingly, a further aspect provides the use of the dietary supplement as described herein for preparing the digestive system of a subject in advance of a weight loss program; for supporting the digestive system of a subject during or after a weight loss program; for supporting fat metabolism, gut barrier function and/or digestive health of a subject; for promoting elimination of toxins from the digestive system of a subject; for avoiding accumulation of toxins in the digestive system of a subject; for supporting liver function and/or kidney function of a subject; for supporting weight loss of a subject; for controlling weight gain; for improving body shape of a subject; for improving waist circumference; and/or for reducing total body fat mass, trunk fat mass, android fat mass and/or energy intake of a subject.

Except when noted, the terms "subject" or "patient" can be used interchangeably and refer to animals, preferably warm-blooded animals, more preferably vertebrates, even more preferably mammals, still more preferably primates, and specifically includes human patients and non-human mammals and primates. Preferred subjects are human subjects. More preferred subjects are adult human subjects. The term "adult" refers to a fully developed and (sexual) mature subject. The threshold of adulthood is typically associated with social and legal concepts. Preferably, the minimal age of the subject entering adulthood is at least 16 years old, at least 17 years old, at least 18 years old, at least 19 years old, at least 20 years old or at least 21 years old, preferably at least 16 years old.

The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, e.g., monkeys and apes. Particularly preferred are human subjects, including both genders and all age categories thereof. More preferred are human adult subjects.

The recitation "in advance of a weight loss program" as used herein refers to a time point or a period before, or previous to, the initiation or start of a weight loss program by a subject. The dietary supplement as described herein may be administered to a subject up to at most 1 week, at most 2 weeks, at most 3 weeks, at most 4 weeks, at most 6 days, at most 5 days, at most 4 days, at most 3 days, at most 2 days, at most 1 day, at most 24 hours, at most 18 hours, at most 12 hours, or at most 6 hours before the initiation of a weight loss program. Preferably, the dietary supplement as described herein is administered to a subject up to at most 24 hours before the initiation or start of a weight loss program.

The term "gut barrier function", "intestinal barrier function" or "gut barrier integrity" as used herein refers to the ability of the digestive system to control absorption of nutrients within the intestine and to defend the body from dangerous macromolecule penetration. The role of the intestines or gut in protecting the mucosal tissues and circulatory system from exposure to pro-inflammatory molecules, such as microorganisms, toxins, and antigens is vital for the maintenance of health and well-being of the subject. The intestinal or gut barrier is a complex system composed of physical, chemical, biological, and immunological barriers. Non-limiting examples of the physical barrier include mucous layer, intestinal epithelial cells, and the tight junctions located at the apical part of it. Non-limiting examples of the chemical barrier include gastric acid, digestive enzyme, and bile acid. Non-limiting examples of the immunological barrier include lymphocytes and immunoglobulin A (Ig A). Non-limiting examples of the biological barrier include normal intestinal flora, the important environmental factor for the energy absorption and storage. Non-limiting examples of factors affecting the intestinal barrier function include flora shift, small intestinal bacterial overgrowth (SIBO), the alteration of tight junction, and increased gut permeability. A disrupted intestinal mucosal barrier can allow passage of microbes, microbial products, and foreign antigens into the mucosa and the body of a subject. This can result in activation of the immune system and secretion of inflammatory mediators. Certain immune responses can subsequently cause cellular damage that can result in further barrier dysfunction. Defects in intestinal mucosal barrier function with the accompanying translocation of microbes and their products have been linked with a variety of health conditions. Non-limiting examples of health conditions in which intestinal mucosal barrier dysfunction is involved include food allergies, microbial infections, irritable bowel syndrome, inflammatory bowel disease, celiac disease, metabolic syndrome, non-alcoholic fatty liver disease, diabetes, and septic shock.

The term "digestive health" as used herein refers to a dynamic process resulting from a digestive system's constant adjustment and adaptation in response to stresses and changes in the environment for maintaining an inner equilibrium (also called homeostasis).

Digestive health may cover the following aspects of the gastrointestinal tract: effective digestion and absorption of food/nutrients; absence of a gastrointestinal illness and/or discomfort; normal and stable intestinal microbiota; effective immune status; a state of well-being; and/or an effective gut barrier function. Non-limiting examples of gastrointestinal illnesses include those of the esophagus, stomach, colon, liver, small intestine, gallbladder, pancreas and biliary tract. Non-limiting examples of gastrointestinal discomfort include bloating, abdominal pain/cramp, and borborygmi. An impaired digestive health may be inherited or develop from multiple factors such as stress, fatigue, diet, alcohol abuse, or smoking.

The term "total body fat mass" as used herein refers to the total amount of fat comprised within the body and includes both essential body fat, which is the minimal amount of fat necessary for normal physiological function, and storage body fat. As certain types of fat mass are more dangerous for health than others, the fat mass of certain body parts may be used to evaluate such health risks. For example, high amounts of abdominal fat, belly fat, trunk fat and/or android fat mass may increase the risk for diabetes. The term "android fat mass" as used herein refers to the adipose tissue which is distributed mainly around the trunk and upper body, in areas such as the abdomen, chest, shoulder and neck. This pattern of fat accumulation may lead to an "apple-shaped" body or central obesity.

The term "body shape" or "silhouette" as used herein refers to the outline of the body of a subject, which is affected by body fat distribution, muscles and bone structure. Body shape may vary due to changes in food habits, average physical exercise and hormone levels. The shape of a subject which has adipose tissue distributed mainly around the trunk and upper body may be referred to as "apple-shaped" body.

The present inventors have further realised that a dietary supplement as defined herein may have a therapeutic effect when administered to subjects having obesity, low-grade inflammation, diabetes, non-alcoholic fatty liver disease (NAFLD), metabolic endotoxemia, metabolic syndrome and/or impaired glucose tolerance.

Therefore, a further aspect provides the use of the dietary supplement as described herein or the sachet as described herein as a nutraceutical.

The term "nutraceutical" as used herein refers to a pharmaceutical-grade and standardized food product that comprises one or more ingredients which provide medical benefits, including the prevention and treatment of disease. A nutraceutical may induce no side effects upon ingestion.

A further aspect provides the dietary supplement as described herein for use in the treatment of obesity, low-grade inflammation, diabetes, non-alcoholic fatty liver disease (NAFLD), metabolic endotoxemia, metabolic syndrome and/or impaired glucose tolerance in a subject.

The terms "treat" or "treatment" encompass both the therapeutic treatment of an already developed disease or condition, such as the therapy of an already developed obesity, low-grade inflammation, diabetes, non-alcoholic fatty liver disease (NAFLD), metabolic endotoxemia, metabolic syndrome and/or impaired glucose tolerance, as well as prophylactic or preventive measures, wherein the aim is to prevent or lessen the chances of incidence of an undesired affliction, such as to prevent occurrence, development and progression of obesity, low-grade inflammation, diabetes, non-alcoholic fatty liver disease (NAFLD), metabolic endotoxemia, metabolic syndrome and/or impaired glucose tolerance. Beneficial or desired clinical results may include, without limitation, alleviation of one or more symptoms, diminishment of extent of disease, and the like.

The recitation "obesity, low-grade inflammation, diabetes, non-alcoholic fatty liver disease (NAFLD), metabolic endotoxemia, metabolic syndrome and/or impaired glucose tolerance" as used herein refers to obesity, low-grade inflammation, diabetes, non-alcoholic fatty liver disease (NAFLD), metabolic endotoxemia, metabolic syndrome, impaired glucose tolerance, or a combination of one or more such as two or more, three or more, or four or more, such as five, six, or seven thereof.

The term "obesity" as used herein refers to an abnormal or excessive body fat accumulation in a subject which may impair health, such as increasing the likelihood of cardiovascular diseases, type 2 diabetes, obstructive sleep apnea, certain types of cancer, osteoarthritis and depression. Obesity is typically linked to body mass index (BMI), which is the most commonly accepted measurement for overweight and/or obesity and is obtained by dividing a person's weight by the square of the person's height. A BMI greater than or equal to 25 is generally considered overweight. Obesity may be defined as a BMI greater than or equal to 30, with a BMI of 35 or more considered as serious comorbidity obesity, and a BMI of 40 or more considered morbid obesity. Non-limiting examples of obesity are obesity, comorbidity obesity and morbid obesity. The term "obese subject" as used herein may be defined as a subject having a BMI of more than or equal to 30.

The term "low-grade inflammation" as used herein refers to an immune system response in a subject which is defined by a 2- to 3-fold increase in plasma concentrations of cytokines, such as TNF-alpha, IL-6 and CRP, and acute phase proteins. Low-grade inflammation may occur when the body sends an inflammatory response to a perceived internal threat that does not require an inflammatory response. Adipose tissue, the liver, muscles and the pancreas may be sites of inflammation in obesity.

The term "diabetes" as used herein refers to all forms of diabetes, which are characterised by disordered metabolism and abnormally high blood sugar resulting from insufficient levels of the hormone insulin in a subject. Non-limiting examples of diabetes are Type 1 diabetes, Type 2 diabetes, gestational diabetes and impaired glucose tolerance. The term "Type 1 diabetes" refers to an autoimmune condition in a subject where the body attacks and destroys insulin-producing cells in the pancreas, meaning no insulin is produced by the body. The term "Type 2 diabetes" refers to a health condition in a subject in which the body doesn't produce enough insulin, or the produced insulin is not used properly by the body, leading to a build-up of glucose in the blood. The term "gestational diabetes" refers to any degree of glucose intolerance with onset or first recognition during pregnancy. The term "impaired glucose tolerance", "IGT" or "prediabetes" as used herein refers to a transition phase between normal glucose tolerance and diabetes in a subject, in which the blood glucose is raised beyond normal blood glucose levels (i.e. 4-8 mmol/L), but not high enough to warrant a diabetes diagnosis. Subjects having impaired glucose tolerance have a greater risk of developing diabetes and cardiovascular disease. According to the criteria of the World Health Organisation (WHO), impaired glucose tolerance is defined as two-hour glucose levels of 140 to 199 mg per dL (7.8 to 11.0 mmol/l) on the 75-g oral glucose tolerance test. A patient is said to be under the condition of IGT when he/she has an intermediately raised glucose level after 2 hours, but less than the level that would qualify for type 2 diabetes mellitus. The fasting glucose may be either normal or mildly elevated.

The term "non-alcoholic fatty liver disease" or "NAFLD" as used herein refers to a type of fatty liver in a subject which occurs when fat is deposited in the liver due to causes other than excessive alcohol use. The main stages of NAFLD are simple fatty liver (steatosis), non-alcoholic steatohepatitis (NASH), fibrosis and cirrhosis. NAFLD may be diagnosed by detecting higher-than-normal levels of liver enzymes in the blood of the patient and/or by detecting an excessive fat content in the liver by ultrasound.

The term "metabolic endotoxemia" as used herein refers to a medical condition characterised by sub-clinically elevated levels of bacterial lipopolysaccharide (LPS) in the bloodstream of a subject. Metabolic endotoxemia may be associated with a heightened pro-inflammatory and oxidant environment often observed in obesity.

The term "metabolic syndrome", "metabolic syndrome X", "syndrome X", "insulin resistance syndrome", "Reaven's syndrome" or "CHAOS" as used herein refers to a combination of medical disorders in a subject that increase the risk of developing cardiovascular disease and diabetes. Metabolic syndrome is typically a clustering of at least three of the five following medical conditions: abdominal (central) obesity, elevated blood pressure, elevated fasting plasma glucose, high serum triglycerides and low high-density lipoprotein (HDL) levels. The World Health Organization (WHO) criteria (1999) for metabolic syndrome require the presence of any one of diabetes mellitus, impaired glucose tolerance, impaired fasting glucose or insulin resistance, and two of the following: (i) blood pressure: ≥ 140/90 mmHg, (ii) dyslipidemia: triglycerides (TG): ≥ 1.695 mmol/L and high-density lipoprotein cholesterol (HDL-C) ≤ 0.9 mmol/L (male), ≤ 1.0 mmol/L (female), (iii) Central obesity: waist:hip ratio > 0.90 (male); > 0.85 (female), or body mass index > 30 kg/m² and (iv) microalbuminuria: urinary albumin excretion ratio ≥ 20 µg/min or albumin:creatinine ratio ≥ 30 mg/g.

A further aspect provides a method of treating obesity, low-grade inflammation, diabetes, non-alcoholic fatty liver disease (NAFLD), metabolic endotoxemia, metabolic syndrome and/or impaired glucose tolerance in a subject in need of such a treatment, comprising administering a therapeutically effective amount of the above defined dietary supplement to the subject.

As used herein, a phrase such as "a subject in need of treatment" includes subjects that would benefit from treatment of a given condition, particularly obesity, low-grade inflammation, diabetes, non-alcoholic fatty liver disease (NAFLD), metabolic endotoxemia, metabolic syndrome and/or impaired glucose tolerance. Such subjects may include, without limitation, those that have been diagnosed with said condition, those prone to develop said condition and/or those in who said condition is to be prevented.

The term "therapeutically effective amount" as used herein, refers to an amount of active ingredient or pharmaceutical agent that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses for the active ingredients as taught herein

In related aspects, the invention provides the use of the above defined dietary supplement for the manufacture of a medicament for the treatment of obesity, low-grade inflammation, diabetes, non-alcoholic fatty liver disease (NAFLD), metabolic endotoxemia, metabolic syndrome and/or impaired glucose tolerance in a subject.

In certain embodiments, the dietary supplement as described herein is to be administered orally.

The recitation "administered orally", "oral administration", "oral delivery", "oral consumption", or "administered per os" as used herein, refers to a route of administration to a subject where a substance or composition is taken through the mouth of said subject. One or more substances taken through the mouth may have a systemic or local (e.g. in the mount, oesophagus, stomach, intestines and/or rectum) effect in the subject. The dietary supplement may be ingested orally in the form of a powder, a swallowable tablet, a sublingual tablet, an effervescent tablet, a chewable tablet, a capsule, a pastille or a liquid preparation. If the dietary supplement is in powder form, it may be solubilized in any type of liquid, for example in water, milk or fruit juice, before administration to the subject.

In certain embodiments, the dietary supplement as described herein is to be administered once a day.

In certain embodiments the dietary supplement as described herein may be administered to a subject at any time of the day, for example before a meal, after a meal, in between meals, during the day, at night, in the morning or in the evening.

In certain embodiments, the dietary supplement as described herein is to be administered orally and once a day.

In certain embodiments, the dietary supplement as described herein may be administered at a dose of from 3000 mg to 15000 mg. For instance, the dietary supplement as described herein may be administered at a dose of from 4000 mg to 12500 mg, from 5000 mg to 12000 mg, from 6000 to 10000 mg, from 7000 mg to 9000 mg, from 7500 mg to 8500 mg, or from 8000 mg to 8500 mg. Preferably, the dietary supplement as described herein may be administered at a dose of from 6000 to 10000 mg. For example, the dietary supplement may administered at a dose of 8000 mg, 8100 mg, 8200 mg, 8300 mg, 8400 mg, or 8500 mg.

In certain embodiments, the dietary supplement as described herein is to be administered orally, once a day, and at a dose of from 6000 mg to 10000 mg.

Another aspect provides a method for producing the dietary supplement as described herein, the method comprising the step of preparing a mixture of: at least one probiotic and at least one ingredient selected from: an ingredient capable of cleansing toxins and protecting against their build up, an ingredient capable of supporting lipid and fatty acid metabolism, an ingredient capable of supporting healthy digestion, wherein said ingredient capable of supporting healthy digestion is an ion or a salt thereof, and an ingredient capable of supporting healthy liver function thereby obtaining the dietary supplement.

In certain embodiments, the method for producing the dietary supplement as described herein comprises the step of preparing a mixture of: at least one probiotic and at least one ingredient selected from:
- an ingredient capable of cleansing toxins and protecting against their build up,
- an ingredient capable of supporting lipid and fatty acid metabolism,
- an ingredient capable of supporting healthy liver function, and
- a salt capable of supporting healthy digestion,
thereby obtaining the dietary supplement.

In certain embodiments, the method for producing the dietary supplement as described herein comprises the step of preparing a mixture of: the probiotic; the chloride or the salt thereof; the choline or the salt thereof; the zinc or the salt thereof; the vitamin B2; and the prebiotic, thereby obtaining the dietary supplement.

In certain embodiments, the method for producing the dietary supplement as described herein comprises the step of preparing a mixture of: the probiotic; the chloride salt; the choline or the salt thereof; the zinc or the salt thereof; the vitamin B2; and the prebiotic, thereby obtaining the dietary supplement.

Another aspect provides a method for producing the dietary supplement as described herein, the method comprising the step of preparing a mixture of: the probiotic; the chloride or the salt thereof; the choline or the salt thereof; the zinc or the salt thereof; the vitamin B2; and the prebiotic, thereby obtaining the dietary supplement.

In certain embodiments, the mixture is a homogeneous mixture.

In certain embodiments, the method for producing the dietary supplement as described herein may further comprise including one or more components selected from the group consisting of flavouring agents, coloring agents, acidyfing agents, and anticaking agents in the mixture.

In certain embodiments, the method for producing the dietary supplement as described herein may further comprise configuring the dietary supplement into an administration form suitable for oral administration.

In certain embodiments, the method for producing the dietary supplement as described herein may comprise configuring the dietary supplement into a powder, a swallowable tablet, a sublingual tablet, an effervescent tablet, a chewable tablet, a capsule, a pastille or a liquid preparation, preferably a powder. The skilled person will understand that depending on the type of dosage form, the method of production and, optionally the type of excipients, will need to be adjusted. For example, to configure the dietary supplement in a tablet, first, a powder or granule mixture may be prepared. Subsequently, a mold may be filled with said mixture, compressed and ejected, thereby obtaining the tablet.

In certain embodiments, the method for producing the dietary supplement as described herein may be performed at ambient temperature (i.e. room temperature). In certain embodiments, the method for producing the dietary supplement as described herein may be performed at a temperature from 10°C to 25°C, from 15°C to 25°C, or from 18°C to 22°C. Preferably, the method for producing the dietary supplement as described herein may be performed at a temperature from 18°C to 25°C.

In certain embodiments, the method for producing the dietary supplement as described herein may be performed at a relative humidity of less than 40%RH or less than 35%RH. Preferably, the method for producing the dietary supplement as described herein may be performed at a relative humidity of less than 30% RH.

In certain embodiments, the method for producing the dietary supplement as described herein may be performed at a temperature from 18°C to 25°C and/or at a relative humidity of less than 30 %RH.

The term "relative humidity" or "RH" as used herein refers to the ratio of the partial pressure of water vapour to the equilibrium vapour pressure of water at a given temperature. Relative humidity depends on temperature and the pressure of the system of interest. The relative humidity may be determined by any means known by the skilled person to measure relative humidity.

Another aspect provides a method for producing the sachet as described herein comprising a step of filling a sachet with a single dose of the dietary supplement as described herein and closing the filled sachet.

In certain embodiments, the sachet is filled and/or sealed in such a way that it allows the sachet to be opened without spilling its content. This may be achieved by filling at most 50%, at most 60%, at most 70%, at most 80% of the volume of said sachet with the dietary supplement as described herein.

In certain embodiments, the filling and/or closing of the sachet is performed in airtight conditions.

In certain embodiments, the filled sachet is closed by sealing.

In certain embodiments, a single dose of the dietary supplement may be from 3000 mg to 15000 mg. For instance, the single dose of the dietary supplement may be from 4000 mg to 12500 mg, from 5000 mg to 12000 mg, from 6000 to 10000 mg, from 7000 mg to 9000 mg, from 7500 mg to 8500 mg, or from 8000 mg to 8500 mg. Preferably, the single dose of the dietary supplement is from 6000 mg to 10000 mg. For example, the single dose of the dietary supplement is 8000 mg, 8100 mg, 8200 mg, 8300 mg, 8400 mg, or 8500 mg.

The present application also provides aspects and embodiments as set forth in the following Statements:
Statement 1. A dietary supplement for weight loss and/or weight maintenance, wherein the dietary supplement comprises: at least one probiotic and at least one ingredient selected from:
   - an ingredient capable of cleansing toxins and protecting against their build up,
   - an ingredient capable of supporting lipid and fatty acid metabolism,
   - an ingredient capable of supporting healthy digestion, wherein said ingredient capable of supporting healthy digestion is an ion or a salt thereof, and
   - an ingredient capable of supporting healthy liver function.
Statement 2. The dietary supplement according to statement 1, wherein
   - the ingredient capable of cleansing toxins and protecting against their build up is selected from choline or a salt thereof, vitamin B or a salt thereof and zinc or a salt thereof;
   - the ingredient capable of supporting lipid and fatty acid metabolism is selected from zinc or a salt thereof, and choline or a salt thereof;
   - the ingredient capable of supporting healthy digestion is chloride or a salt thereof; and/or
   - the ingredient capable of supporting healthy liver function is choline or a salt thereof.
Statement 3. The dietary supplement according to statement 1 or 2, comprising a probiotic; chloride or a salt thereof; choline or a salt thereof; zinc or a salt thereof; vitamin B2; and a prebiotic.
Statement 4. A dietary supplement (for weight loss and/or weight maintenance) comprising: a probiotic; chloride or a salt thereof; choline or a salt thereof; zinc or a salt thereof; vitamin B2; and a prebiotic.
Statement 5. The dietary supplement according to any one of statements 1 to 4, wherein the probiotic is selected from the group consisting of Bifidobacterium, Saccharomyces, Debaromyces, Candida, Pichia, Torulopsis, Aspergillus, Rhizopus, Mucor, Penicillium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, and Lactobacillus, preferably wherein the probiotic is Bifidobacterium.
Statement 6. The dietary supplement according to any one of statements 1 to 5, wherein the probiotic is one or more Bifidobacterium species selected from the group consisting of *Bifidobacterium animalis*, *Bifidobacterium lactis, Bifidobacterium bifidium*, *Bifidobacterium longum, Bifidobacterium breve, Bifidobacterium infantis, Bifidobacterium catenulatum*, *Bifidobacterium adolescentis*, and *Bifidobacterium angalatum*.
Statement 7. The dietary supplement according to any one of statements 1 to 6, wherein the probiotic is a Bifidobacterium of the species *Bifidobacterium animalis*.
Statement 8. The dietary supplement according to any one of statements 1 to 7, wherein the probiotic is a Bifidobacterium of the species *Bifidobacterium animalis* subsp. *lactis* or *Bifidobacterium animalis* subsp. *animalis* or a combination thereof.
Statement 9. The dietary supplement according to any one of statements 1 to 8, wherein the probiotic is a Bifidobacterium of the species *Bifidobacterium animalis* subsp. *lactis.*
Statement 10. The dietary supplement according to any of statements 1 to 9, wherein the prebiotic is a soluble food fiber.
Statement 11. The dietary supplement according to any of statements 1 to 10, wherein the prebiotic is one or more soluble food fibers selected from the group consisting of maltodextrin, inulin, oligofructose, lactose, lactulose, resistant starch, fructooligosaccharides (FOS), galactooligosaccharides (GOS), transgalactooligosaccharides (TOS), wheat bran-derived arabinoxylooligosaccharides (AXOS), xylooligosaccharides (XOS), isomaltooligosaccharides (IMO), gentiooligosaccharides (GTO), glucooligosaccharides, pectic oligosaccharides, soy oligosaccharides, lactosucrose, polydextrose, sugar alcohols, alpha glucan, beta glucan, wheat dextrin, guar gum, flaxseed gum, fenugreek gum, acacia gum, psyllium, and raffinose.
Statement 12. The dietary supplement according to any of statements 3 to 11, wherein the prebiotic is maltodextrin.
Statement 13. The dietary supplement according to any of statements 3 to 12, comprising from 10⁶ to 10¹⁴ CFU of the probiotic per 100 g of said dietary supplement; from 0.01 to 35 percent by weight of chloride or the salt thereof; from 0.01 to 6 percent by weight of choline or the salt thereof; from 0.001 to 0.25 percent by weight of zinc or the salt thereof; from 0.0005 to 0.02 percent by weight of vitamin B2; and/or from 1 to 95 percent by weight of said prebiotic;
Statement 14. The dietary supplement according to any of statements 3 to 13, comprising from 10⁸ to 10¹² CFU of the probiotic per 100 g of said dietary supplement; from 0.9 to 2.2 percent by weight of chloride or the salt thereof; from 0.8 to 1.5 percent by weight of choline or the salt thereof; from 0.01 to 0.02 percent by weight of zinc or the salt thereof; from 0.002 to 0.004 percent by weight of vitamin B2; and/or from 58 to 88 percent by weight of said prebiotic.
Statement 15. The dietary supplement according to any of statements 3 to 14, in which a daily serving of said dietary supplement comprises from 10⁶ to 10¹⁴ CFU of said probiotic; from 1 mg to 3000 mg of chloride or the salt thereof; from 1 mg to 500 mg of choline or the salt thereof; from 0. 08 mg to 20 mg of zinc or the salt thereof; from 0.04 mg to 2 mg of vitamin B2; and/or from 100 mg to 15000 mg of said prebiotic.
Statement 16. The dietary supplement according to any of statements 3 to 15, in which a daily serving of said dietary supplement comprises from 10⁸ to 10¹² CFU of said probiotic; from 75 mg to 175 mg of chloride or the salt thereof; from 65mg to 125 mg of choline or the salt thereof; from 0.8 mg to 1.7 mg of zinc or the salt thereof; from 0.1 mg to 0.4 mg of vitamin B2; and/or from 4500 mg to 7500 mg of said prebiotic.
Statement 17. The dietary supplement according to any one of statements 1 to 16, wherein the dietary supplement is stable for at least 24 months when stored at 4°C degrees or at room temperature.
Statement 18. The dietary supplement according to any one of statements 1 to 17, wherein the dietary supplement retains at least 1.22 x 10⁹ CFU of the probiotic per gram of said dietary supplement for at least 24 months when stored at a temperature from 10°C to 25°C.
Statement 19. The dietary supplement according to any one of statements 1 to 18, wherein the dietary supplement comprises one or more components selected from the group consisting of flavouring agents, coloring agents, acidifying agents, and anticaking agents.
Statement 20. The dietary supplement according to any one of statements 1 to 19, wherein said dietary supplement comprises no fatty acids and/or no carbohydrates in addition to fatty acids and/or carbohydrates comprised within the prebiotic and/or probiotic.
Statement 21. The dietary supplement according to any one of statements 1 to 20, wherein the dietary supplement is configured for oral administration.
Statement 22. The dietary supplement according to any one of statements 1 to 21, wherein the dietary supplement is in the form of a powder, a swallowable tablet, a sublingual tablet, an effervescent tablet, a chewable tablet, a capsule, a pastille, a liquid preparation, preferably a powder.
Statement 23. Use of the dietary supplement according to any one of statements 1 to 22, for preparing the digestive system of a subject in advance of a weight loss program; for supporting the digestive system of a subject during or after a weight loss program; for supporting fat metabolism, gut barrier function and/or digestive health of a subject; for promoting elimination of toxins from the digestive system of a subject; for avoiding accumulation of toxins in the digestive system of a subject; for supporting liver function and/or kidney function of a subject; for supporting weight loss of a subject; for improving body shape of a subject; and/or for reducing total body fat mass, trunk fat mass, android fat mass and/or energy intake of a subject.
Statement 24. Use of the dietary supplement according to any one of statements 1 to 22 as a nutraceutical.
Statement 25. The dietary supplement according to any one of statements 1 to 22, for use in a method of treating obesity, low-grade inflammation, diabetes, non-alcoholic fatty liver disease (NAFLD), metabolic endotoxemia, metabolic syndrome and/or impaired glucose tolerance in a subject.
Statement 26. The dietary supplement for use according to statement 24 or 25, wherein said dietary supplement is to be administered orally.
Statement 27. The dietary supplement for use according to statement 25 or 26, wherein said dietary supplement is to be administered once a day.
Statement 28. The dietary supplement for use according to any one of statements 25 to 27, wherein said dietary supplement is to be administered at a dose of 6000 to 10 000 mg.
Statement 29. A sachet comprising the dietary supplement according to any one of statements 1 to 22.
Statement 30. The sachet according to statement 29, wherein the sachet comprises 6000 to 10 000 mg of the dietary supplement.
Statement 31. A method for producing the dietary supplement according to any one of statements 1 to 22, the method comprising the step of preparing a mixture of: at least one probiotic and at least one ingredient selected from:
   - an ingredient capable of cleansing toxins and protecting against their build up,
   - an ingredient capable of supporting lipid and fatty acid metabolism,
   - an ingredient capable of supporting healthy digestion, wherein said ingredient capable of supporting healthy digestion is an ion or a salt thereof,, and
   - an ingredient capable of supporting healthy liver function
   thereby obtaining the dietary supplement.
Statement 32. The method according to statement 30, wherein the method comprises the step of preparing a mixture of: the probiotic; the chloride or the salt thereof; the choline or the salt thereof; the zinc or the salt thereof; the vitamin B2; and the prebiotic, thereby obtaining the dietary supplement.
Statement 33. A method for producing the dietary supplement according to any one of statements 4 to 22, the method comprising the step of preparing a mixture of: the probiotic; the chloride or the salt thereof; the choline or the salt thereof; the zinc or the salt thereof; the vitamin B2; and the prebiotic, thereby obtaining the dietary supplement.
Statement 34. The method according to any one of statements 31 to 33, further comprising including one or more components selected from the group consisting of flavouring agents, coloring agents, acidifying agents, and anticaking agents in the mixture.
Statement 35. The method according to any one of statements 31 to 34, further comprising configuring the dietary supplement into an administration form suitable for oral administration.
Statement 36. The method according to any one of statements 31 to 35, wherein said method is performed at a temperature of between 18 and 25°C and/or a relative humidity of less than 30 %RH.
Statement 37. A method for producing the sachet according to statement 29 or 30 comprising a step of filling a sachet with a single dose of the dietary supplement according to any one of statements 1 to 22 and closing the filled sachet.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The herein disclosed aspects and embodiments of the invention are further supported by the following non-limiting examples.

### EXAMPLES

### Example 1: Dietary supplement according to an embodiment of the present invention

The composition of a dietary supplement according to an embodiment of the present invention is given in Table 1. Table 1 lists the ingredients and the amount of each ingredient in the dietary supplement according to an embodiment of the present invention.

**Table 1: Dietary supplement according to an embodiment of the present invention**

| **Ingredients** | **Commercial name** | **Supplier** | **Amount** |
|---|---|---|---|
| *Bifidobacterium animalis* subsp. *lactis* | Howaru B420 | Dupont NV | 10¹⁰ CFU |
| Maltodextrin | Fibersol-2 | C.F.M. | 6027.8 mg |
| Potassium chloride | Potassio Cloruro C.A. | Polichimica | 120 mg |
| Choline bitartrate (i.e. silicon dioxide) | Choline bitartrate DAB-USP | A.C.E.F. | 82.5 mg |
| Zinc gluconate | Zinco gluconato | Giusto Faravelli | 1.5 mg |
| Vitamin B2 (i.e. Riboflavine) | Riboflavin High Flow 100 | BASF | 0210 mg |
| Additional ingredients (e.g. flavour, acidifying agent, anticaking agent, etc.) | Silarom Lemon, Syloid 244 FP, Acido citrico | Silesia, Grace, Citrique Belge | up to 8200 mg |
| Total amount | | | 8200 mg |

### Example 2: administration of the dietary supplement of example 1 to a subject prior to a weight-loss program

8200 mg of the dietary supplement of Example 1 in a powder form is dissolved in water or another liquid (for example a smoothie or fruit juice) and administered to a subject once a day for a period of 1 to 3 months. Immediately hereafter, the subject enrolls into a weight loss program.

The subject's body fat mass, trunk fat mass, waist circumference, body weight, energy intake, body shape, liver function, kidney function, gut barrier function, fat metabolism, blood glucose, BMI, inflammatory status and/or cholesterol levels, are evaluated before the period in which the subject is given the dietary supplement, immediately after said period (i.e. just before the weight loss program) and after the weight loss program.

### Example 3: stability evaluation of the dietary supplement of example 1

The stability and/or shelf life of the dietary supplement according to an embodiment of the invention (example 1) was evaluated by analysing the time of the organoleptic, microbiological and nutritional features of the supplement. The evaluation was conducted in line with the International Council for Harmonisation (IHC) guidelines.

Two different conditions of conservation/storage were analysed. A first condition ("sample A") wherein the dietary supplement according to an embodiment of the invention was conserved at 25°C and 60% RH during 24 months and a second condition ("sample B") wherein the dietary supplement according to an embodiment of the invention was conserved at 30°C and 75% RH during 6 months (see Table 1).

**Table 1:**

| **SAMPLE** | **CONSERVATION TEMPERATURE** | **TEST DURATION** |
|---|---|---|
| A | 25°C +/- 2°C/60%RH | 24 Months |
| B | 30°C +/- 2°C/75%RH | 6 Months |

| | | |
|---|---|---|
| **Instrumentation:** ICP-OES mod. OPTIMA 8000, Aw meter TESTO 650, Liquid chromatograph HPLC + UV detector Jasco | | |

The results of the stability analysis, including the physical and organoleptic analysis, the microbiological analysis and the chemical analysis, obtained for sample A and B during the first two months of conservation are shown below. "C" indicates that the results were conform with the IHC guidelines, "NC" indicates that the results were not conform with the IHC guidelines. The physical and organoleptic analysis is also performed after 3 months, 6 months, 9 months, 12 months, 18 months, and 24 months. Microbiological analysis is also performed at the time points indicated below. Chemical analysis is also performed after 3 months, 6 months, 12 months, 18 months, and 24 months.

### Physical and organoleptic analysis

**Analysis: Water activity (aw)**

| | **Sample** | | | **Result** | | |
|---|---|---|---|---|---|---|
| **Time** | **A** | **B** | **Range** | **A** | **B** | **MU** |
| 0 Months | 0,166 | / | <=0,2 | C | / | -- |
| 2 Months | / | 0,126 | <=0,2 | / | C | -- |

**Analysis: Appearance**

| | **Sample** | | | **Result** | | |
|---|---|---|---|---|---|---|
| **Time** | **A** | **B** | **Range** | **A** | **B** | **MU** |
| 0 Months | powder | / | powder | C | / | -- |
| 2 Months | / | powder | powder | / | C | -- |

**Analysis: Colour**

| | **Sample** | | | **Result** | | |
|---|---|---|---|---|---|---|
| **Time** | **A** | **B** | **Range** | **A** | **B** | **MU** |
| 0 Months | white | / | white | C | / | -- |
| 2 Months | / | white | white | / | C | -- |

**Analysis: Smell**

| | **Sample** | | | **Result** | | |
|---|---|---|---|---|---|---|
| **Time** | **A** | **B** | **Range** | **A** | **B** | **MU** |
| 0 Months | typical | / | typical | C | / | -- |
| 2 Months | / | typical | typical | / | C | -- |

**Analysis: Taste**

| | **Sample** | | | **Result** | | |
|---|---|---|---|---|---|---|
| **Time** | **A** | **B** | **Range** | **A** | **B** | **MU** |
| 0 Months | typical | / | typical | C | / | -- |
| 2 Months | / | typical | typical | / | C | -- |

### Microbiological analysis

**Analysis: ANALISI B.LACTIS**

| | **Sample** | | | **Result** | | |
|---|---|---|---|---|---|---|
| **Time** | **A** | **B** | **Range** | **A** | **B** | **MU** |
| 0 Months | 6,64x10⁹ | / | >=1,22*10⁹ | C | / | CFU/g |
| 2 Months | / | 4,45*10⁹ | >=1,22*10⁹ | / | C | CFU/g |

ANALISI B.LACTIS is measured after 3 months, 6 months, 12 months, 18 months, and 24 months.

**Analysis: Total plate count**

| | **Sample** | | | **Result** | | |
|---|---|---|---|---|---|---|
| **Time** | **A** | **B** | **Range** | **A** | **B** | **MU** |
| 0 Months | <10³ | / | <=10⁴ | C | / | CFU/g |
| 2 Months | / | <10³ | <=10⁴ | / | C | CFU/g |

Total plate count is measured after 3 months, 6 months, 12 months, 18 months, and 24 months.

**Analysis: Escherichia coli**

| | **Sample** | | | **Result** | | |
|---|---|---|---|---|---|---|
| **Time** | **A** | **B** | **Range** | **A** | **B** | **MU** |
| 0 Months | absent | / | absent | C | / | CFU/g |

Escherichia coli is measured after 6 months and 24 months.

**Analysis: Enterobacters**

| | **Sample** | | | **Result** | | |
|---|---|---|---|---|---|---|
| **Time** | **A** | **B** | **Range** | **A** | **B** | **MU** |
| 0 Months | <10² | / | <=10² | C | / | CFU/g |
| 2 Months | / | <10² | <=10² | / | C | CFU/g |

Enterobacters is measured after 3 months, 6 months, 12 months, 18 months, and 24 months.

**Analysis: Staphylococcus aureus**

| | **Sample** | | | **Result** | | |
|---|---|---|---|---|---|---|
| **Time** | **A** | **B** | **Range** | **A** | **B** | **MU** |
| 0 Months | absent | / | absent | C | / | CFU/g |

Staphylococcus aureus is measured after 6 months and 24 months.

**Analysis: Yeast/Moulds**

| | **Sample** | | | **Result** | | |
|---|---|---|---|---|---|---|
| **Time** | **A** | **B** | **Range** | **A** | **B** | **MU** |
| 0 Months | <10² | / | <=10² | C | / | CFU/g |
| 2 Months | / | <10² | <=10² | / | C | CFU/g |

Yeast/Moulds is measured after 3 months, 6 months, 12 months, 18 months, and 24 months.

**Analysis: Salmonella**

| | **Sample** | | | **Result** | | |
|---|---|---|---|---|---|---|
| **Time** | **A** | **B** | **Range** | **A** | **B** | **MU** |
| 0 Months | absent | / | absent | C | / | CFU/25 g |

Salmonella is measured after 6 months and 24 months.

### Chemical analysis

**Analysis: Vitamin B2**

| | **Sample** | | | **Result** | | |
|---|---|---|---|---|---|---|
| **Time** | **A** | **B** | **Range** | **A** | **B** | **MU** |
| 0 Months | 0,21 | / | 0,17 - 0,32 | C | / | mg/sachet |
| 2 Months | / | in corso | 0,17 - 0,32 | / | | mg/sachet |

**Analysis: Zinc**

| | **Sample** | | | **Result** | | |
|---|---|---|---|---|---|---|
| **Time** | **A** | **B** | **Range** | **A** | **B** | **MU** |
| 0 Months | 1,23 | / | 1,2 - 2,18 | C | / | mg/sachet |
| 2 Months | / | 1,56 | 1,2 - 2,18 | / | C | mg/sachet |

**Analysis: Dietary fiber including resistant maltodextrin**

| | **Sample** | | | | **Result** | | |
|---|---|---|---|---|---|---|---|
| **Time** | **A** | **B** | **Range** | **Date** | **A** | **B** | **MU** |
| 0 Months | 6,29 | / | 4,8 - 7,28 | 01/08/17 | C | / | g/sachet |
| 2 Months | / | 6,52 | 4,8 - 7,28 | 02/10/17 | / | C | g/sachet |

**Analysis: Chlorine**

| | **Sample** | | | **Result** | | |
|---|---|---|---|---|---|---|
| **Time** | **A** | **B** | **Range** | **A** | **B** | **MU** |
| 0 Months | 98,4 | / | 80 - 174 | C | / | mg/sachet |
| 2 Months | / | 131,2 | 80 - 174 | / | C | mg/sachet |

**Analysis: Choline**

| | **Sample** | | | **Result** | | |
|---|---|---|---|---|---|---|
| **Time** | **A** | **B** | **Range** | **A** | **B** | **MU** |
| 0 Months | 103,3 | / | 66 - 123,75 | C | / | mg/sachet |
| 2 Months | / | 98,4 | 66 - 123,75 | / | C | mg/sachet |

## Claims

1. A dietary supplement for weight loss and/or weight maintenance, wherein the dietary supplement comprises: a probiotic; a chloride salt; choline or a salt thereof; zinc or a salt thereof; vitamin B2; and a prebiotic, wherein said dietary supplement comprises no fatty acids and/or no carbohydrates in addition to fatty acids and/or carbohydrates comprised within the prebiotic and/or probiotic.

2. The dietary supplement according to claim 1, wherein the probiotic is selected from the group consisting of Bifidobacterium, Saccharomyces, Debaromyces, Candida, Pichia, Torulopsis, Aspergillus, Rhizopus, Mucor, Penicillium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, and Lactobacillus; preferably wherein the probiotic is one or more Bifidobacterium species selected from the group consisting of *Bifidobacterium animalis*, *Bifidobacterium lactis, Bifidobacterium bifidium*, *Bifidobacterium longum, Bifidobacterium breve, Bifidobacterium infantis, Bifidobacterium catenulatum*, *Bifidobacterium adolescentis*, and *Bifidobacterium angalatum*; more preferably wherein the probiotic is a Bifidobacterium of the species *Bifidobacterium animalis*; even more preferably wherein the probiotic is a Bifidobacterium of the species *Bifidobacterium animalis* subsp. *lactis* or *Bifidobacterium animalis* subsp. *animalis* or a combination thereof; yet more preferably wherein the probiotic is a Bifidobacterium of the species *Bifidobacterium animalis* subsp. *lactis.*

3. The dietary supplement according to claim 1 or 2, wherein the prebiotic is a soluble food fiber; preferably wherein the prebiotic is one or more soluble food fibers selected from the group consisting of maltodextrin, inulin, oligofructose, lactose, lactulose, resistant starch, fructooligosaccharides (FOS), galactooligosaccharides (GOS), transgalactooligosaccharides (TOS), wheat bran-derived arabinoxylooligosaccharides (AXOS), xylooligosaccharides (XOS), isomaltooligosaccharides (IMO), gentiooligosaccharides (GTO), glucooligosaccharides, pectic oligosaccharides, soy oligosaccharides, lactosucrose, polydextrose, sugar alcohols, alpha glucan, beta glucan, wheat dextrin, guar gum, flaxseed gum, fenugreek gum, acacia gum, psyllium, and raffinose; more preferably wherein the prebiotic is maltodextrin.

4. The dietary supplement according to any of claims 1 to 3, comprising from 10⁶ to 10¹⁴ CFU of the probiotic per 100 g of said dietary supplement; from 0.01 to 35 percent by weight of a chloride salt; from 0.01 to 6 percent by weight of choline or the salt thereof; from 0.001 to 0.25 percent by weight of zinc or the salt thereof; from 0.0005 to 0.02 percent by weight of vitamin B2; and/or from 1 to 95 percent by weight of said prebiotic; or comprising from 10⁸ to 10¹² CFU of the probiotic per 100 g of said dietary supplement; from 0.9 to 2.2 percent by weight of a chloride salt; from 0.8 to 1.5 percent by weight of choline or the salt thereof; from 0.01 to 0.02 percent by weight of zinc or the salt thereof; from 0.002 to 0.004 percent by weight of vitamin B2; and/or from 55 to 90 percent by weight of said prebiotic.

5. The dietary supplement according to any of claims 1 to 4, in which a daily serving of said dietary supplement comprises from 10⁶ to 10¹⁴ CFU of said probiotic; from 1 mg to 3000 mg of a chloride salt; from 1 mg to 500 mg of choline or the salt thereof; from 0.08 mg to 20 mg of zinc or the salt thereof; from 0.04 mg to 2 mg of vitamin B2; and/or from 100 mg to 15000 mg of said prebiotic; or in which a daily serving of said dietary supplement comprises from 10⁸ to 10¹² CFU of said probiotic; from 75 mg to 175 mg of a chloride salt; from 65mg to 125 mg of choline or the salt thereof; from 0.8 mg to 1.7 mg of zinc or the salt thereof; from 0.1 mg to 0.4 mg of vitamin B2; and/or from 4500 mg to 7500 mg of said prebiotic.

6. The dietary supplement according to any one of claims 1 to 5, wherein the dietary supplement is stable for at least 24 months when stored at 4°C degrees or at room temperature and/or wherein the dietary supplement retains at least 1.22 x 10⁹ CFU of the probiotic per gram of said dietary supplement, when being stored for at least 24 months when stored at a temperature from 10°C to 25°C.

7. The dietary supplement according to any one of claims 1 to 6, wherein the dietary supplement comprises one or more components selected from the group consisting of flavouring agents, coloring agents, acidifying agents, and anticaking agents.

8. The dietary supplement according to any one of claims 1 to 7, wherein the dietary supplement is in the form of a powder, a swallowable tablet, a sublingual tablet, an effervescent tablet, a chewable tablet, a capsule, a pastille, a liquid preparation, preferably a powder.

9. Use of the dietary supplement according to any one of claims 1 to 8, for preparing the digestive system of a subject in advance of a weight loss program; for supporting the digestive system of a subject during or after a weight loss program; for supporting fat metabolism, gut barrier function and/or digestive health of a subject; for promoting elimination of toxins from the digestive system of a subject; for avoiding accumulation of toxins in the digestive system of a subject; for supporting liver function and/or kidney function of a subject; for supporting weight loss of a subject; for improving body shape of a subject; and/or for reducing total body fat mass, trunk fat mass, android fat mass and/or energy intake of a subject.

10. Use of the dietary supplement according to any one of claims 1 to 8 as a nutraceutical.

11. The dietary supplement according to any one of claims 1 to 8, for use in a method of treating obesity, low-grade inflammation, diabetes, non-alcoholic fatty liver disease (NAFLD), metabolic endotoxemia, metabolic syndrome and/or impaired glucose tolerance in a subject.

12. The dietary supplement for use according to claim 11, wherein said dietary supplement is to be administered once a day and/or wherein said dietary supplement is to be administered at a dose of 6000 to 10 000 mg.

13. A sachet comprising the dietary supplement according to any one of claims 1 to 8.

14. The sachet according to claim 13, wherein the sachet comprises 6000 to 10 000 mg of the dietary supplement.

15. A method for producing the dietary supplement according to any one of claims 1 to 8, the method comprising the step of preparing a mixture of: the probiotic; the chloride salt; the choline or the salt thereof; the zinc or the salt thereof; the vitamin B2; and the prebiotic, thereby obtaining the dietary supplement.

16. The method according to claim 15, further comprising including one or more components selected from the group consisting of flavouring agents, coloring agents, acidifying agents, and anticaking agents, in the mixture and/or further comprising configuring the dietary supplement into an administration form suitable for oral administration.

17. The method according to claim 15 or 16, wherein said method is performed at a temperature from 18°C to25°C and/or at a relative humidity of less than 30% RH.

18. A method for producing the sachet according to claim 13 or 14 comprising a step of filling a sachet with a single dose of the dietary supplement according to any one of claims 1 to 8 and closing the filled sachet.
